Europäisches Patentamt

European Patent Office

Office européen des brevets

(19)

(11) Publication number: 0 597 110 A1

## EUROPEAN PATENT APPLICATION
### published in accordance with Art. 158(3) EPC

(12)

(21) Application number: 93906349.1

(22) Date of filing: 09.10.92

(86) International application number:
PCT/JP92/01317

(87) International publication number:
WO 93/07487 (15.04.93 93/10)

(51) Int. Cl.5: **G01N 33/569**, C12P 21/02,
C12P 21/08, C12N 15/10,
C12N 15/31, C07K 13/00,
//(C12N15/31,C12R1:445)

(30) Priority: **09.10.91 JP 290742/91**
**23.04.92 JP 129956/92**

(43) Date of publication of application:
**18.05.94 Bulletin 94/20**

(84) Designated Contracting States:
**DE FR GB**

(71) Applicant: **IATRON LABORATORIES, INC.**
**11-4 Higashi-Kanda 1-chome**
**Chiyoda-ku Tokyo 101(JP)**

(72) Inventor: **KAMIO, Yoshiyuki**
**1-1, Sumiyoshidai-higashi 2-chome,**
**Izurni-ku**
**Sendai-shi, Miyagi 981-32(JP)**
Inventor: **IZAKI, Kazuo**
**15-14, Higashi-Koyodai 2-chome,**
**Tomiya-machi**
**Kurokawa-gun, Miyagi 981-33(JP)**
Inventor: **KITA, Hiroshi, Iatron Laboratories,**
**Inc.**
**11-4, Higashi-kanda 1-chome**
**Chiyoda-ku, Tokyo 101(JP)**

(74) Representative: **VOSSIUS & PARTNER**
**Postfach 86 07 67**
**D-81634 München (DE)**

(54) METHOD OF DETECTING MESITYLENE-RESISTANT STAPHYLOCOCCUS AUREUS, NOVEL PEPTIDE, AND DNA CODING FOR SAME.

(57) A method of detecting mesitylene-resistant *Staphylococcus aureus* (MRSA) with an antibody against proleucocidin or leucocidin; a DNA which codes for proleucocidin or leucocidin and a process for producing the DNA; a process for producing proleucocidin or leucocidin by using the DNA; and proleucocidin or leucocidin produced thereby. MRSA can be detected rapidly and accurately.

EP 0 597 110 A1

*Fig. 5*

TECHNICAL FIELD

The present invention relates to novel peptides specifically produced by methicillin resistant *Staphylococcus aureus* (hereinafter also referred to as "MRSA"), DNAs encoding the above peptides, and a method for detecting MRSA using an antibody against the above peptides.

BACKGROUND ART

*Staphylococcus aureus* is a facultative anaerobic Grampositive coccus. *S. aureus* is naturally and widely distributed and causes nosocomial and opportunistic infection. It is considered as an important pathogen which causes pyelitis, cystitis, impetigo, circumscribed pyocephalus, osteomyelitis, septicemia, etc. Of the *Staphylococcus aureus*, there exists a methicillin resistant *Staphylococcus aureus* (MRSA) which acquired a high degree of resistance against various antibiotics due to chromosomal mutation. The MRSA proliferates in resistless persons, such as an aged person, a newborn baby, or a cancer patient to cause pneumonia, septicemia or the like, and in some cases result in death. Therefore, it is clinically very important to effectively diagnose MRSA infection.

For the determination of MRSA, isolation of microorganisms from body fluids (such as blood, urine, saliva, etc.) to identify the same as MRSA, or a sensitivity test to antibodies, was carried out. In the conventional methods as above, however, troublesome procedures were required for definite determination. In spite of the demands to accurately judge the MRSA infection in an early stage with a high sensitivity, no satisfactory methods existed.

The present inventors found that the existence of peptides specifically produced by MRSA, and thus succeeded in cloning the DNAs encoding the peptides (that is, leukocidin) and their premature proteins (proleukocidin), using oligonucleotide probes corresponding to the partial amino acid sequence of leukocidin, and finally succeeded in expression of leukocidins S and F and proleukocidins S and F, using the above cloned DNAs. Furthermore, the present inventors prepared antibodies against leukocidins S and F and proleukocidins S and F, and the detection of MRSA was attempted with the antibodies. As a result, it was found to detect MRSA quickly, accurately, and specifically. The present invention is based upon the above findings.

DISCLOSURE OF THE INVENTION

Accordingly, the present invention relates to a peptide having an amino acid sequence of the formula:

```
Ala Asn Asp Thr Glu Asp Ile Gly Lys Gly Ser Asp Ile Glu Ile Ile
Lys Arg Thr Glu Asp Lys Thr Ser Asn Lys Trp Gly Val Thr Gln Asn
Ile Gln Phe Asp Phe Val Lys Asp Thr Lys Tyr Asn Lys Asp Ala Leu
Ile Leu Lys Met Gln Gly Phe Ile Ser Ser Arg Thr Thr Tyr Tyr Asn
Tyr Lys Lys Thr Asn His Val Lys Ala Met Arg Trp Pro Phe Gln Tyr
Asn Ile Gly Leu Lys Thr Asn Asp Lys Tyr Val Ser Leu Ile Asn Tyr
Leu Pro Lys Asn Lys Ile Glu Ser Thr Asn Val Ser Gln Thr Leu Gly
Tyr Asn Ile Gly Gly Asn Phe Gln Ser Ala Pro Ser Leu Gly Gly Asn
Gly Ser Phe Asn Tyr Ser Lys Ser Ile Ser Tyr Thr Gln Gln Asn Tyr
Val Ser Glu Val Glu Gln Gln Asn Ser Lys Ser Val Leu Trp Gly Val
Lys Ala Asn Ser Phe Ala Thr Glu Ser Gly Gln   Lys Ser Ala Phe Asp
Ser Asp Leu Phe Val Gly Tyr Lys Pro His Ser Lys Asp Pro Arg Asp
Tyr Phe Val Pro Asp Ser Glu Leu Pro Pro Leu Val Gln Ser Gly Phe
Asn Pro Ser Phe Ile Ala Thr Val Ser His Glu Lys Gly Ser Ser Asp
Thr Ser Glu Phe Glu Ile Thr Tyr Gly Arg Asn Met Asp Val Thr His
Ala Ile Lys Arg Ser Thr His Tyr Gly Asn Ser Tyr Leu Asp Gly His
Arg Val His Asn Ala Phe Val Asn Arg Asn Tyr Thr Val Lys Tyr Glu
Val Asn Trp Lys Thr His Glu Ile Lys Val Lys Gly Gln Asn;
```

and a DNA containing a base sequence encoding the above peptide.
    Further, the present invention relates to a peptide having an amino acid sequence of the formula:

```
Met Leu Lys Asn Lys Ile Leu Ala Thr Thr Leu Ser Val Ser Leu Leu
Ala Pro Leu Ala Asn Pro Leu Leu Glu Asn Ala Lys Ala Ala Asn Asp
Thr Glu Asp Ile Gly Lys Gly Ser Asp Ile Glu Ile Ile Lys Arg Thr
Glu Asp Lys Thr Ser Asn Lys Trp Gly Val Thr Gln Asn Ile Gln Phe
Asp Phe Val Lys Asp Thr Lys Tyr Asn Lys Asp Ala Leu Ile Leu Lys
```

```
Met Gln Gly Phe Ile Ser Ser Arg Thr Thr Tyr Tyr Asn Tyr Lys Lys
Thr Asn His Val Lys Ala Met Arg Trp Pro Phe Gln Tyr Asn Ile Gly
Leu Lys Thr Asn Asp Lys Tyr Val Ser Leu Ile Asn Tyr Leu Pro Lys
Asn Lys Ile Glu Ser Thr Asn Val Ser Gln Thr Leu Gly Tyr Asn Ile
Gly Gly Asn Phe Gln Ser Ala Pro Ser Leu Gly Gly Asn Gly Ser Phe
Asn Tyr Ser Lys Ser Ile Ser Tyr Thr Gln Gln Asn Tyr Val Ser Glu
Val Glu Gln Gln Asn Ser Lys Ser Val Leu Trp Gly Val Lys Ala Asn
Ser Phe Ala Thr Glu Ser Gly Gln Lys Ser Ala Phe Asp Ser Asp Leu
Phe Val Gly Tyr Lys Pro His Ser Lys Asp Pro Arg Asp Tyr Phe Val
Pro Asp Ser Glu Leu Pro Pro Leu Val Gln Ser Gly Phe Asn Pro Ser
Phe Ile Ala Thr Val Ser His Glu Lys Gly Ser Ser Asp Thr Ser Glu
Phe Glu Ile Thr Tyr Gly Arg Asn Met Asp Val Thr His Ala Ile Lys
Arg Ser Thr His Tyr Gly Asn Ser Tyr Leu Asp Gly His Arg Val His
Asn Ala Phe Val Asn Arg Asn Tyr Thr Val Lys Tyr Glu Val Asn Trp
Lys Thr His Glu Ile Lys Val Lys Gly Gln Asn;
```

and a DNA containing a base sequence encoding the above peptide.

Still further, the present invention relates to a peptide having an amino acid sequence of the formula:

```
Ala Glu Gly Lys Ile Thr Pro Val Ser Val Lys Lys Val Asp Asp Lys
Val Thr Leu Tyr Lys Thr Thr Ala Thr Ala Asp Ser Asp Lys Phe Lys
Ile Ser Gln Ile Leu Thr Phe Asn Phe Ile Lys Asp Lys Ser Tyr Asp
Lys Asp Thr Leu Val Leu Lys Ala Thr Gly Asn Ile Asn Ser Gly Phe
Val Lys Pro Asn Pro Asn Asp Tyr Asp Phe Ser Lys Leu Tyr Trp Gly
Ala Lys Tyr Asn Val Ser Ile Ser Ser Gln Ser Asn Asp Ser Val Asn
Ala Val Asp Tyr Ala Pro Lys Asn Gln Asn Glu Glu Phe Gln Val Gln
Asn Thr Leu Gly Tyr Thr Phe Gly Gly Asp Ile Ser Ile Ser Asn Gly
Leu Ser Gly Gly Leu Asn Gly Asn Thr Ala Phe Ser Glu Thr Ile Asn
Tyr Lys Gln Glu Ser Tyr Arg Thr Leu Ser Arg Asn Thr Asn Tyr Lys
Asn Val Gly Trp Gly Val Glu Ala His Lys Ile Met Asn Gly Trp Gly
Pro Tyr Gly Arg Asp Ser Phe His Pro Thr Tyr Gly Asn Glu Leu Phe
Leu Ala Gly Arg Gln Ser Ser Ala Tyr Ala Gly Gln Asn Phe Ile Ala
Gln His Gln Met Pro Leu Leu Ser Arg Ser Asn Phe Asn Pro Arg Phe
Leu Ser Val Leu Ser His Arg Gln Asp Ala Ala Lys Lys Ser Lys Ile
Thr Val Thr Tyr Gln Arg Glu Met Asp Leu Tyr Gln Ile Arg Trp Asn
Gly Phe Tyr Trp Ala Gly Ala Asn Tyr Lys Asn Phe Lys Thr Arg Thr
Phe Lys Ser Thr Tyr Glu Ile Asp Trp Glu Asn His Lys Val Lys Leu
Leu Asp Thr Lys Glu Thr Glu Asn Asn Lys;
```

and a DNA containing a base sequence encoding the above peptide.

Also, the present invention relates to a peptide having an amino acid sequence of the formula:

```
Met Lys Met Asn Lys Leu Val Lys Ser Ser Val Ala Thr Ser Met Ala
Leu Leu Leu Leu Ser Gly Thr Ala Asn Ala Glu Gly Lys Ile Thr Pro
Val Ser Val Lys Lys Val Asp Asp Lys Val Thr Leu Tyr Lys Thr Thr
Ala Thr Ala Asp Ser Asp Lys Phe Lys Ile Ser Gln Ile Leu Thr Phe
Asn Phe Ile Lys Asp Lys Ser Tyr Asp Lys Asp Thr Leu Val Leu Lys
Ala Thr Gly Asn Ile Asn Ser Gly Phe Val Lys Pro Asn Pro Asn Asp
Tyr Asp Phe Ser Lys Leu Tyr Trp Gly Ala Lys Tyr Asn Val Ser Ile
Ser Ser Gln Ser Asn Asp Ser Val Asn Ala Val Asp Tyr Ala Pro Lys
Asn Gln Asn Glu Glu Phe Gln Val Gln Asn Thr Leu Gly Tyr Thr Phe
Gly Gly Asp Ile Ser Ile Ser Asn Gly Leu Ser Gly Gly Leu Asn Gly
Asn Thr Ala Phe Ser Glu Thr Ile Asn Tyr Lys Gln Glu Ser Tyr Arg
Thr Leu Ser Arg Asn Thr Asn Tyr Lys Asn Val Gly Trp Gly Val Glu
Ala His Lys Ile Met Asn Gly Trp Gly Pro Tyr Gly Arg Asp Ser Phe
His Pro Thr Tyr Gly Asn Glu Leu Phe Leu Ala Gly Arg Gln Ser Ser
Ala Tyr Ala Gly Gln Asn Phe Ile Ala Gln His Gln Met Pro Leu Leu
Ser Arg Ser Asn Phe Asn Pro Arg Phe Leu Ser Val Leu Ser His Arg
Gln Asp Ala Ala Lys Lys Ser Lys Ile Thr Val Thr Tyr Gln Arg Glu
Met Asp Leu Tyr Gln Ile Arg Trp Asn Gly Phe Tyr Trp Ala Gly Ala
Asn Tyr Lys Asn Phe Lys Thr Arg Thr Phe Lys Ser Thr Tyr Glu Ile
Asp Trp Glu Asn His Lys Val Lys Leu Leu Asp Thr Lys Glu Thr Glu
Asn Asn Lys;
```

and a DNA containing a base sequence encoding the above peptide.

In addition, the present invention relates to a method for preparing a DNA encoding leukocidin or proleukocidin characterized by isolating a chromosomal DNA from methicillin resistant *Staphylococcus aureus*, digesting the DNA with a restriction enzyme, inserting the resulting DNA fragment into a vector, transforming a host by the vector to prepare a DNA library, using a synthetic DNA encoding a partial amino acid sequence of leukocidin or proleukocidin purified from the methicillin resistant *Staphylococcus aureus* as a probe to select a DNA encoding proleukocidin or proleukocidin from said DNA library, and cloning the resulting DNA.

Further, the present invention relates to a method for preparing leukocidin or proleukocidin characterized by ligating a DNA encoding leukocidin or proleukocidin with an expression vector to prepare a reproducible recombinant DNA, transforming a host with the recombinant DNA, cultivating the transformant to express the DNA encoding leukocidin or proleukocidin to produce leukocidin or proleukocidin, and isolating the resulting leukocidin or proleukocidin.

Still further, the present invention relates to a method for detecting methicillin resistant *Staphylococcus aureus* characterized in that an antibody for proleukocidin or leukocidin is used. In the base sequence of the present specification, A is an adenine residue, C is a cytosine residue, G is a guanine residue, and T is a thymidine residue.

BRIEF DESCRIPTION OF THE DRAWINGS

Figure 1 is a graph showing the results of purification of S-component of leukocidin from MRSA strain No. 4 by HPLC equipped with a TSK gel SP-5PW column. The S-component was determined by immunoblotting (the shadowed area in Fig. 1 is the S-component).

Figure 2 shows the results of an SDS-PAGE pattern of the purified S-component preparation from MRSA strain No. 4 and *Staphylococcus aureus* V8. Lane 1 is the S-component from *Staphylococcus aureus* V8, lane 2 is the S-component from MRSA strain No. 4, and lane 3 is the molecular weight marker.

6

The molecular weight markers used are as follows:

1... phosphorylase (97.4 kDa)

2... bovine serum albumin (66.2 kDa)

3... ovalbumin (45 kDa)

4... bovine carbonic anhydrase (31 kDa)

5... soybean trypsin inhibitor (21.5 kDa)

6... lysozyme (14.4 kDa)

Figure 3 illustrates the state where a HindIII-XbaI fragment (2.2 kb) is inserted in a plasmid vector pUC119. The abbreviations in Fig. 3 are as follows:

H... HindIII; E... EcoRI; X.. XbaI; B... BamHI; K... KpnI.

Figure 4 is a restriction map of a HindIII-XbaI fragment (2.2 kb). The leukocidin (S-component) coding region is shown by an arrow. The double line portion is the inserted 2.2 kb fragment. The single line portion is the polylinker region of the vector plasmid pUC119. The abbreviations are as follows:

H... HindIII; E... EcoRI; X.. XbaI; B... BamHI; K... KpnI.

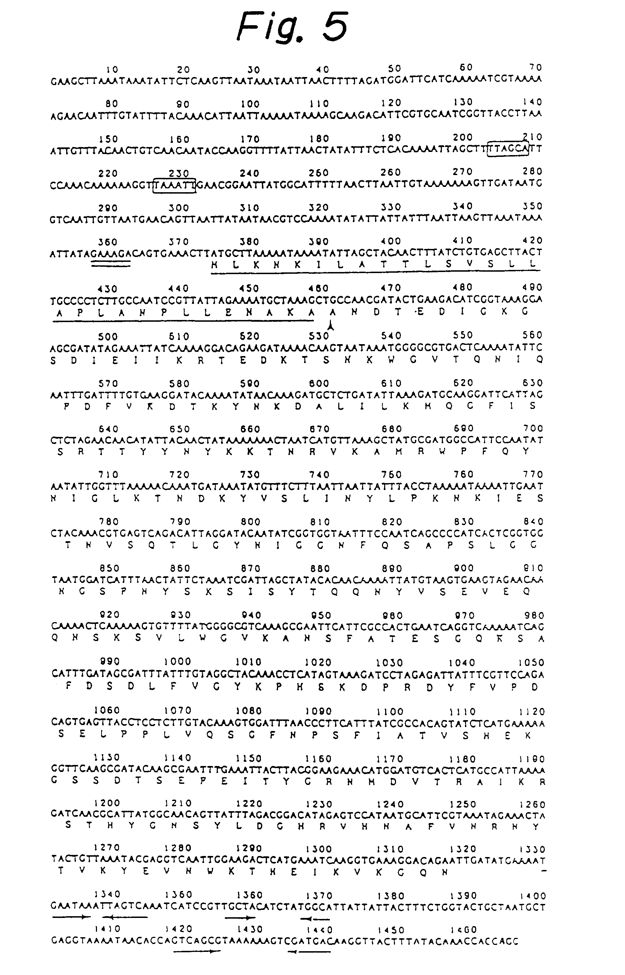

Figure 5 shows the base sequence of the leukocidin (S-component) coding region and the amino acid sequence of the leukocidin (S-component) deduced therefrom. The sequence is arranged so that the first (1) is the first nucleotide in the recognition base sequence for the restriction enzyme HindIII and the 1466th is the last nucleotide in pSRK91-1DNA. A putative ribosomal binding site is indicated by double underlines. A putative promoter site is indicated by rectangular boxes. Three inverted repeats downstream of the translation termination codon are indicated by horizontal arrows. A signal sequence is indicated by a single underline. Vertical arrow represents the processing site of the prematured S-component.

Figure 6 shows the result of the electrophoresis of the *in vitro* translation product encoded by the plasmid pSRK91. Lane 1 is the *in vitro* product encoded by the pSRK91 DNA, lane 2 is the *in vitro* product encoded by pUC119, and PS is the prematured S-component.

Figure 7 shows the results of Western blotting. In (A), Lane 1 is the purified S-component, lane 2 is the cell extract of *Escherichia coli* (pSRK91), lane 3 is the cell extract of *Escherichia coli* (pUC119), lane 4 is the culture medium from *Escherichia coli* (pSRK91), and lane 5 is the culture medium from *Escherichia coli* (pUC119).

(B) shows the results of analysis of the S-component in the cell extract from *Escherichia coli* DH5$\alpha$ - (pSRK91). Lane 1 is the result in the presence of IPTG, lane 2 is the result in the absence of IPTG, and lane 3 is the standard S-component.

Figure 8 shows the results of Western blotting of the cell fraction of *Escherichia coli* DH5$\alpha$ (pSRK91). Lane 1 is the standard S protein, lane 2 is the shocked cell fraction, lane 3 is a periplasmic fraction, lane 4 is the total cell fraction, and lane 5 is the culture medium.

Figure 9 is a graph showing the results of purification of the F-component of leukocidin from MRSA strain No. 4 by HPLC equipped with a TSK gel SP-5PW column. The F-component was determined by immunoblotting. Hatched area in Fig. 9 is F-component.

Figure 10 shows the results of the SDS-PAGE pattern of purified F-component preparation from MRSA strain No. 4 and *Staphylococcus aureus* V8. Lane 1 is the F-component from the *Staphylococcus aureus* V8, lane 2 is the F-component from MRSA strain No. 4, and lane 3 is a molecular weight marker. The molecular weight markers used are the same as those of Fig. 2.

Figure 11 illustrates the state where an EcoRI-EcoRI fragment (4.0 kb) is inserted into the plasmid vector pUC119. The abbreviations in Fig. 11 have the meanings same as those of Fig. 3, except that C means ClaI.

Figure 12 is a restriction map of an EcoRI-EcoRI fraction (4.0 kb). The leukocidin (F-component) coding region is shown by the thick arrow F. The double line portion is the 4.0 kb fragment which is inserted. The single line portion is the polylinker region of the vector plasmid pUC119. The abbreviations are the same as those of Fig. 4, except that C means ClaI.

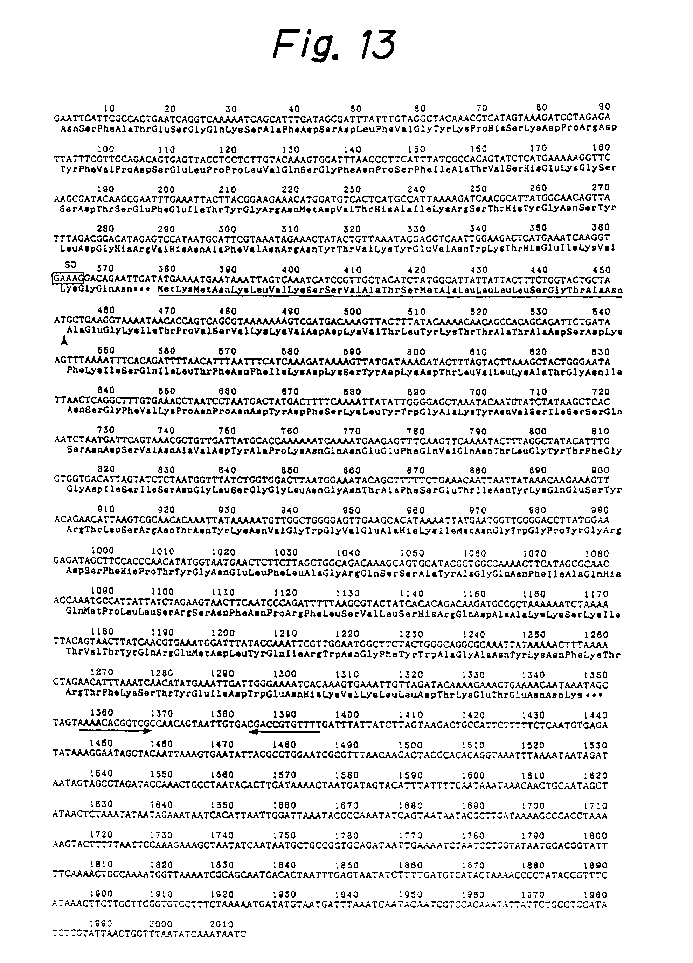

Figure 13 shows the base sequence of the leukocidin (F-component) coding region and the amino acid sequence of the leukocidin (F-component) deduced therefrom. The sequence is arranged so that the first (1) is the first nucleotide of the recognition base sequence in the restriction enzyme EcoRI and the 2012nd is the last nucleotide in pFRK92 DNA. A putative promoter site is indicated by a rectangular box. an inverted repeat downstream of he translation termination codon is indicated by horizontal arrows. A signal sequence is indicated by a single underline. Vertical arrow represents the processing site of the prematured F-component.

Figure 14 shows the result of the electrophoresis of the *in vitro* translation product encoded by the plasmid pFRK92. Lane 1 is the *in vitro* product encoded by the pFRK92 DNA, lane 2 is the *in vitro* product encoded by pUC119, and PS is the prematured F-component.

Figure 15 shows the results of Western blotting. Lane 2 is the purified F-component, lane 1 is the cell extract of *Escherichia coli* (pFRK92), lane 3 is the cell extract of *Escherichia coli* (pUC119), lane 4 is the culture medium from *Escherichia coli* (pFRK92), and lane 5 is the culture medium from *Escherichia coli* - (pUC119).

Figure 16 shows the results of Western blotting of the cell fraction of *Escherichia coli* DH5α (pFRK92). Lane 1 is the standard F protein, lane 3 is the shocked cell fraction, and lane 2 is a periplasmic fraction.

BEST MODE FOR CARRYING OUT THE INVENTION

A cytopathic exotoxin produced by *Staphylococcus aureus* comprises two protein fractions [that is, the F- (fast eluted) component which quickly emerges from the column and the S- (slow eluted) component which is retained on the column longer than the F-component when isolated by cation exchange chromatography]. It was known that the S-component and the F-component are inert when separating from each other, but act synergistically to induce cytotoxic changes in human and rabbit polymorphonuclear leukocytes. However, there was no information about the relation between the chemical structure and the function of each component. As explained in detail below, the present inventors found with the aid of a gene manipulation technique that the S-component is a protein (matured leukocidin) comprising 286 amino acids, which is derived from a prematured protein (proleukocidin) comprising 315 amino acids and produced within a cell. Further, it was also found that the F-component is a protein (matured leukocidin) comprising 298 amino acids, which is derived from a prematured protein (proleukocidin) comprising 323 amino acids and produced within a cell. The cloning of the DNA encoding leukocidin and proleukocidin, and the expression of the DNA will be explained hereinafter.

(A) Purification of Leukocidin S- and F-Components and Elucidation of Partial Amino Acid Sequences Thereof

The S- and L-components of the leukocidin are contained in the supernatant of the culture fluid of methicillin resistant *Staphylococcus aureus* (MRSA). The leukocidin S- and F-components can be obtained by adding zinc chloride to the culture supernatant to precipitate, treating the precipitate with sodium phosphate, and purifying the resulting crude product by ion exchange chromatography. The partial amino acid sequence of the resulting leukocidin S- and F-components can be elucidated by, for example, the Edman degradation method, etc.

(B) Synthesis of Probes

Probes can be synthesized by selecting appropriate portions of the partial amino acid sequence determined in the above-mentioned (A). To synthesize the DNA used as the probes, known methods may be used, for example, the phosphoamidite method utilizing an automatic DNA synthesizer, Japan Society of Biochemistry ed., *Genetic Research Methods I*, 1-27 (1986); or Matteucci et al., *Tetrahedron Lett.*, 21, 719 (1980).

(C) Extraction of DNA

DNA is extracted from methicillin resistant *Staphylococcus aureus* (MRSA) which produces and secretes leukocidin (S- and F-components) into the culture supernatant. The chromosomal DNA is extracted by treating the cells with lysostaphin in 25 mM Tris buffer (pH 7.9) and 50 mM glucose, followed by the elution with SDS. The extracted DNA may be purified according to the method of Maniatis et al. [Maniatis et al.: *Molecular Cloning*, A *Laboratory Manual*, Cold Spring Harbor Laboratory, New York (1982)].

(D) Insertion of DNA Into Vector

The method of Maniatis et al. may be used to insert the DNA into the vector, for example, pUC or pBR vectors.

(E) Screening

The probes synthesized in the above section (B) may be used to screen colonies containing the target gene from a DNA library prepared in the above section (D). First, the colonies are baked on a filter

EP 0 597 110 A1

membrane such as nylon membrane or nitrocellulose. On the other hand, the probes obtained in section (B) are labeled with radioactive isotopes such as ($^{32}$P) by the method described in the above reference of Maniatis et al. The plaques containing the target gene can be screened by hybridizing the plaques baked on the filter membrane and the probes labeled by $^{32}$P etc. Further, it is possible to use the method described in Glover: *DNA Cloning*, 1, 51-52, IRL Press for the screening of the plaques containing the target gene. That is, the presence of the target DNA may be screened either by detecting the activity of the protein expressed from the target DNA or by detecting the protein expressed from the target DNA using an antibody specific to the protein.

(F) Subcloning

As the vectors for the subcloning, there may be mentioned, for example, pUC or pBR plasmids. From the plaques or colonies selected by the above screening, phage DNA or plasmid DNA is extracted, purified, digested with an appropriate restriction enzyme, and inserted into a subcloning vector. The resulting recombinant vector is introduced into a competent cells by the method described, for example, in Hanahan et al.: *Mol. Biol.* 166, 557-580 (1983). As the host cell, there may be mentioned *Escherichia coli*, for example, those stemmed from the *Escherichia coli* K12 strain, *Bacillus subtilis*, or yeast. The resulting transformant may be selected by the method described in the above-mentioned reference of Maniatis et al., for example, the method using isopropyl thiogalactosidase (IPTG).

(G) Preparation of Plasmid DNA

It is possible to purify plasmid DNA from the clone obtained by subcloning, for example, by the alkali-SDS method described in the above-mentioned reference of Maniatis et al. or the boiling method. If necessary, the cesium chloride ultracentrifugation method may also be used.

(H) Structural Analysis of DNA

The plasmid DNA obtained in section (G) is cleaved by various restriction enzymes to prepare a restriction map. Further, the dideoxy method [Sanger et al.: *J. Mol. Biol.* 143, 161-178 (1980)] or the like is used to determine the nucleotide sequence.

(I) Expression

The plasmid DNA obtained in the above section (G) can be used to transform competent cells of the *Escherichia coli* YA21 strain and express leukocidin by the method described, for example, in the above-mentioned reference of Maniatis et al. As the host cells for the expression, there may be mentioned *Escherichia coli*, *Bacillus subtilis*, or yeast, for example, *Escherichia coli* MM294, DH1, DH5, JM109, HB101, GC508, or CES201, in addition to the above-mentioned *Escherichia coli* YA21.

Further, as the vectors which may be used in the present invention, there may be mentioned pUC or pBR vectors which belong to a group of the Co1E1 plasmid vector. Further, as the phage vectors, there may be mentioned the λ phages, such as λgt10, λgt11, Charon 4A, λgtWES.λB, EMBL 3, EMBL 4, etc. Further, as the yeast expression vectors, there may be mentioned, for example, pYES2.0, pAH9, pMAC561, pLG669, pMA91, pAM82 pMC2010, pOP, pTE432, pSD922, etc. As the expression vectors for *Bacillus subtilis*, there may be mentioned, for example, pPL608, pKTH50, pKTH51, pKTH53, pKTH38, pHY300, pLK, etc., and as the expression vectors for the animal cell (for example, COS-7, Bowes melanoma, CHO cells), there may be mentioned, for example, pMT, pSV, pCD, pMDSG, pBPV, etc.

The resulting transformant is cultured in an appropriate known medium until the cell density reaches a sufficient concentration. Then, the cells are ultrasonically treated to disrupt them. The disrupted cells are, for example, centrifuged to obtain the supernatant containing proteins and nucleic acids, etc. Leukocidin is purified from the supernatant by a known method.

The purified leukocidin or proleukocidin may be used as an immunogen to prepare antibodies, that is, the antiserum or monoclonal antibody, by a known method, for example, the method described in *Later Lectures on Biochemical Experiment* "Immunobiochemical Research Methods" (Japan Society of Biochemistry ed.) etc. These antibodies may be labeled by a known method, if necessary. For example, when the proleukocidin or leukocidin is detected by the Western blot hybridization method, a nonradioactive labeled antibody (for example, an enzyme-labeled antibody, biotinized antibody, digoxigeninized antibody, or antibody labeled with a chemofluorescent substance or fluorescent substance) may be used.

9

The liquid sample which may be used in the assay of the present invention is not particularly limited so long as it is suspected to contain methicillin resistant *Staphylococcus aureus*. For example, it is possible to use blood, saliva, urine, immobilized free cells, tissue sections, etc.

When detecting MRSA by the method of the present invention, the liquid sample may be examined directly by the immunological means or the like, using the above antibody. The examination may be effected after the liquid sample is diluted by an appropriate diluent or after the liquid sample is cultured to obtain a supernatant. For example, identification may be carried out by separating the test sample by the SDS-polyacrylamide gel electrophoresis method; electrically absorbing on a nitrocellulose filter; and using a labeled primary antibody or a labeled secondary antibody (so-called Western blotting method). Alternatively, an agglutination reaction by an antigen-antibody reaction may also be used. In this case, an insoluble carrier such as polystyrene is sensitized with the antibody (so-called latex agglutination reaction). Further, the present invention may be applied to a known immunoassay system, such as ELISA, EIA, CLIA, etc. In these cases, detection may be easily performed by visually detecting a signal or by an optical means. From the results, it is possible to diagnose the MRSA infection.

Examples

The present invention now will be further illustrated by, but by no means limited to, the following examples:

Example 1: Purification of MRSA Marker Proteins

A culture medium [containing 250 g of yeast extract (Oriental Yeast Industry), 200 g of cazamino acid (Difco), 200 g of sodium glycerophosphate (Wako Pure Chemical Industries), 60 g of $Na_2HPO_4$-$12H_2O$, 4 g of $KH_2PO_4$, and 0.1% sodium lactate (Wako Pure Chemical Industries) in 10 liters of medium] (hereinafter also referred to as the "medium A") was inoculated with MRSA strain No. 4 [deposited in Research Institute for Microbial diseases, Osaka University under RIMD3109025 (MRSA No. 4)]. MRSA strain No. 4 was cultured therein at 37°C under constant shaking. After 22 hours, the cultured medium was centrifuged at 4°C for 20 minutes at 11,000 x g to remove microbial cells and obtain 10 liters of supernatant. To the supernatant, zinc chloride was added so that the final concentration thereof became 0.1M. Thereafter, the whole was agitated. The resulting precipitate was collected by centrifuging at 4°C for 15 minutes at 8000 x g. The resulting precipitate was suspended in 1 liter of 0.4M sodium phosphate buffer (pH 6.5). The resulting precipitate of zinc phosphate was removed by centrifuging at 4°C for 20 minutes at 10,000 x g. The supernatant was dialyzed against 20 liters of 0.05M acetate buffer (pH 5.2) containing 0.2M sodium chloride (hereinafter also referred to as the "buffer A"), and then ammonium sulfate was added to the saturation and the whole was allowed to stand under agitation for 60 minutes. The resulting turbid portions were collected by centrifuging at 4°C for 20 minutes at 15,000 x g. The resulting precipitate was suspended in the buffer A and dialyzed against the same buffer A. After the dialysis, the resulting solution was placed onto the CM-Sephadex C50 column (2.5 x 50 cm) which had been equilibrated with the buffer A, and then the absorbed protein was eluted by a linear gradient of sodium chloride using 500 ml of the buffer A and 500 ml of 0.05M acetate buffer (pH 5.2) containing 1.2M sodium chloride (hereinafter also referred to as the "buffer B"). Each of the S-component eluted at a 0.9M sodium chloride and the F-component eluted at a 0.5M sodium chloride was collected and dialyzed against the buffer A, respectively.

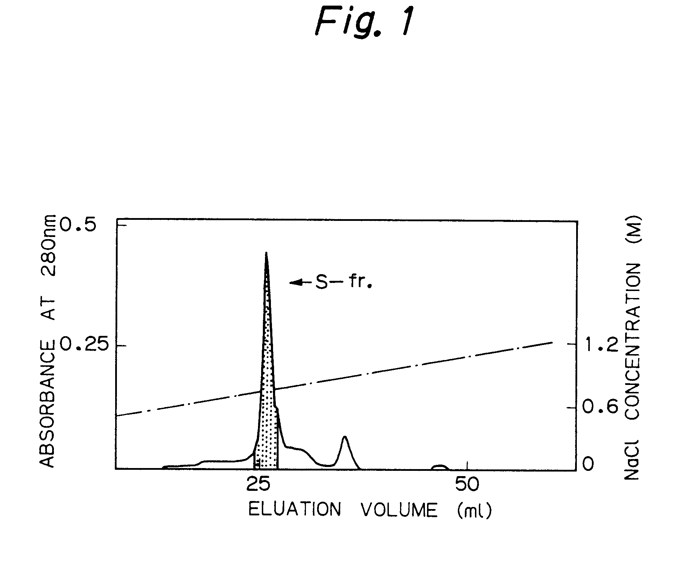

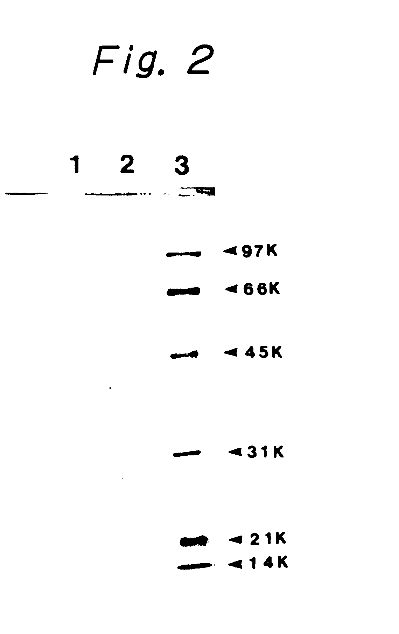

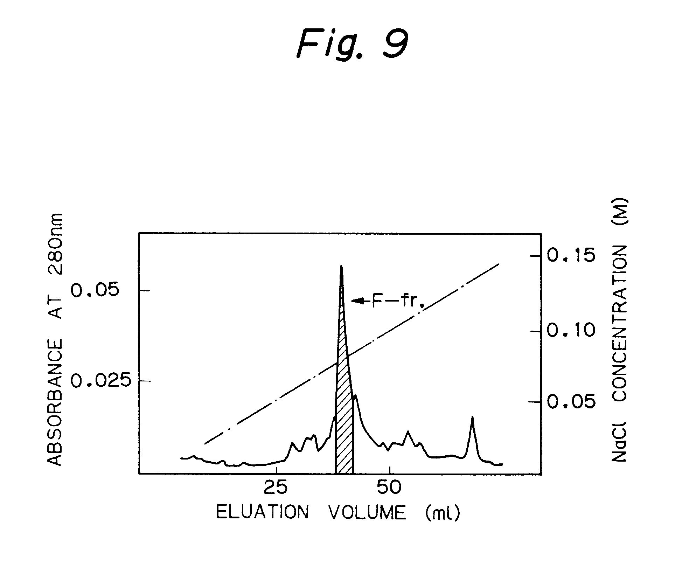

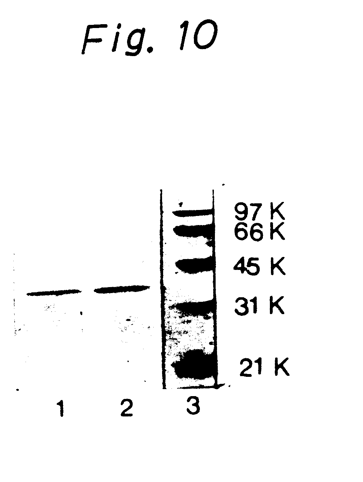

The dialyzed S- and F-components were placed respectively onto columns (0.75 x 7.5 cm) packed with TSK SP-5PW gel and were purified by the linear gradient of sodium chloride using 25 ml of 0.05M acetate buffer (pH 5.2) containing 0.4M sodium chloride and the same amount of the buffer B. The S-component eluted at 0.75M sodium chloride and the F-component eluted at 0.06 to 0.08M sodium chloride were collected. The results are shown in Fig. 1 (S-component) and Fig. 9 (F-component). The resulting marker proteins of MRSA, the S- and the F-components (shadowed area of Fig. 1 and hatched area of Fig. 9) were electrophoretically pure, respectively. The S- and F-components prepared from *Staphylococcus aureus* V8 (ATCC27733), a reference strain of MRSA, in the same manner as above, were placed in lanes 1 of Figs. 2 and 10, whereas the S-and F-components prepared from Example 1 were placed in lanes 2.

Example 2: Primary Structure of Marker Proteins of MRSA

The primary structures of the marker proteins of MRSA obtained in Example 1 were determined by the Edman degradation with a Protein Sequencer Model 6600 (Millipore Milligen Biosearch), whereby it was found that the S-component had an amino acid sequence (from the amino terminus to the 50th amino acid residue) of the following formula (1), and the F-component had an amino acid sequence (from the amino terminus to the 41st amino acid residue) of the following formula (2):

```
Ala Asn Asp Thr Glu Asp Ile Gly Lys Gly Ser Asp Ile Glu Ile Ile
Lys Arg Thr Glu Asp Lys Thr Ser Asn Lys Trp Gly Val Thr Gln Asn
Ile Gln Phe Asp Phe Val Lys Asp Thr Lys Tyr Asn Lys Asp Ala Leu
Ile Leu                                                       (1);

Ala Glu Gly Lys Ile Thr Pro Val Ser Val Lys Lys Val Asp Asp Lys
Val Thr Leu Tyr Lys Thr Thr Ala Thr Ala Asp Ser Asp Lys Phe Lys
Ile Ser Glu Ile Leu Thr Phe Asn Phe                           (2).
```

From the biological activities of the S- and the F-components (assayed by the methods described in Pretlow et al., *Immunology*, 24, 85-92, 1973; Rahman et al., *Biochem. Biophys. Res. Commun.*, 181, 138-144, 1991), the molecular weights of the purified S- and F-components (see Figs. 2 and 10), and the amino acid compositions as shown above, the MRSA marker proteins obtained in Example 1 were identified as leukocidin.

Example 3: Synthesis of Probes

Probes were synthesized to clone the DNAs of the leukocidin. The synthesis was carried out by an ordinary method using a 381 automatic DNA synthesizer Model (Applied Biosystem). For the S-component, the 53 mer DNA of the following formula (3) corresponding to the amino acid sequence determined in Example 2 from the amino terminus to the 18th amino acid residue, the 47 mer DNA of the following formula (4) corresponding to the sequence from the 25th to 40th amino acid residues, and the 20 mer DNA of the following formula (5) corresponding to the sequence from the 25th to 31st amino acid residues were synthesized. For the F-component, the 17 mer DNA of the following formula (6) corresponding to the amino acid sequence determined in Example 2 from the 10th to 15h amino acid residues and the 18 mer DNA of the following formula (7) corresponding to the sequence from the 16th to 21st amino acid residues were synthesized. The probes were used in the form of a mixture.. In the following formulas, "I" denotes an inosine residue, and the 5' end is placed to the left and the 3' end to the right.

```
GCI AAT(or C) GAT(or C) ACI GAA(or G) GAT(or C) ATI GGI AAA(or
G) GGI A(or T)G(or C)I GAT(or C) ATI GAA(or G) ATI ATI AAA(or G)
AG                                                            (3);

AAT(or C) AAA(or G) TGG GGI GTI ACI CAA(or G) AAT(or C) ATI
CAA(or G) TTT(or C) GAT(or C) TTT(or C) GTI AAA(or G) GA     (4);
```

AAT(or C) AAA(or G) TGG GGI GTI ACI CA     (5);
GTI AAA(or G) AAA(or G) GTI GAT(or C) GA     (6);
AAA(or G) GTI ACI C(or T)TI TAT(or C) AAA(or G)     (7).

Example 4: Extraction and Purification of MRSA DNAs

The MRSA strain No. 4 used in Example 1 was cultured under the conditions described in Example 1 and the cells were harvested. The resulting cells were suspended in a 10-fold amount of 10 mM Tris buffer (pH 7.5) and treated with lysostaphin (Sigma) (50 $\mu$l/ml). Then, to solubilize the treated product, a 10% sodium dedecyl sulfate (hereinafter also referred to as SDS) and 10 mM Tris(hydroxymethyl)aminomethane HCl buffer (pH 7.9) containing 10 mM EDTA (hereinafter also referred to as the "Tris buffer 1") were added in an amount of a 0.1 volume of the total volume. To the solubilized solution, a riponuclease (Sigma) was added so that a final concentration thereof became 2 $\mu$g/ml. The whole was maintained at 37°C for one hour, and then protease K was added so that a final concentration thereof became 50 $\mu$g/ml, and digested at 37°C for 2 hours. From the protease-digested liquor, MRSA DNA was extracted from a phenol/water/chloroform solvent. Ethyl alcohol was added to the aqueous layer to precipitate the DNA, and then the precipitated chromosomal DNA was collected onto a glass rod. The resulting DNA was dissolved in a Tris buffer (pH 7.6) containing 1 nM EDTA (hereinafter also referred to as the "Tris buffer 3") and used as an MRSA-DNA standard in the following experiments.

Example 5: Coupling of MRSA DNA Fragments with Plasmid DNAs

The MRSA-DNA (5 $\mu$l; 15 $\mu$g/ml) obtained in Example 4 was digested at 37°C for 4 hours in 20 $\mu$l of a reaction solution comprising in distilled water 2 $\mu$l of a 100 mM Tris buffer (pH 7.5) containing a restriction enzyme HindIII (20 units), 100 mM-MgCl$_2$, and 10 mM dithiothreitol, and then partially digested at 37°C for 10 minutes in 20 $\mu$l of a reaction solution comprising in distilled water 2 $\mu$l of a Tris buffer (pH 7.5) containing the restriction enzyme XbaI (20 units), 100 mM MgCl2, 10 mM dithiothreitol, 50 mM sodium chloride, and 0.1% bovine serum albumin (hereinafter also referred to as "BSA"), to thereby obtain the MRSA-DNA fragments (S-component). The DNA fragments of the F-component were prepared by repeating the same procedure, except that the restriction enzyme EcoRI (20 units) was used.

For the coupling of the DNA fragments with plasmid, restriction enzymes XbaI and HindIII for S component and EcoRI for F component were used under the same conditions. To pUC119 plasmid DNA (Takara Shuzo; 19.5 $\mu$l), 1.0 $\mu$l of 3M sodium chloride, 2.5 $\mu$l of 0.1M Tris buffer (pH 8.0), and 2 $\mu$l of bacterial alkali phosphatase (Takara Shuzo; *Escherichia coli* A19) (hereinafter also referred to as "BAP") were added, and the pUC119 plasmid DNA was treated therein at 65°C for 1 hour. The BAP-treated pUC119 plasmid DNA was extracted under the conditions described in Example 4 after adding the Tris buffer 3 (25 $\mu$l) and 3M sodium acetate (15 $\mu$l) to the reaction liquor. Ethyl alcohol was added to precipitate. The resulting precipitate was dried at room temperature for 30 minutes, and then dissolved in 30 $\mu$l of distilled water. The solution A (8 parts by volume) and the solution B (1 part by volume) of DNA ligase (Takara DNA Ligation Kit) were added to a mixture of 0.45 $\mu$g MRSA-DNA fragment prepared by the above method and 0.15 $\mu$g (8.74 $\mu$l) of BAP-treated pUC119 plasmid DNA and the whole was allowed to react at 16°C overnight. The reaction solution after the completion of the reaction was designated as the solution I.

Example 6: Transformation of Bacteria by Plasmids pSRK91 and pFRK92

A 2XYT medium (5 ml) [having a composition of 10 g/liter of trypton, 10 g/liter of yeast extract, and 5 g/liter of sodium chloride] was inoculated with a platinum loopful amount of ampillicin sensitive *Escherichia coli* DH5$\alpha$ (Nippon Gene). The *E. coli* DH5$\alpha$ was cultured at 37°C overnight. The resulting cultured medium (360 $\mu$l) was transferred to 36 ml of a fresh 2XYT medium, and then culturing was continued under constant shaking at 37°C until the turbidity thereof at 600 nm reached to 0.4. The resulting culture medium (25 ml) was taken, and the cells were harvested therefrom by centrifugation and then suspended in cold 50 mM CaCl$_2$ (12.5 ml). The suspension was designated as the solution II.

The solution I (20 $\mu$l) prepared in Example 5 and the above solution II (200 $\mu$l) were mixed and allowed to stand at 0 to 5°C for 30 minutes. Thereafter, the temperature was raised to 42°C and the mixture was allowed to stand for further 45 seconds. Then, 780 $\mu$l of Luria-Bertani medium (pH 7.5) preheated at 37°C and having a composition of 10 g/liter of ampillicin-containing trypton, 5 g/liter of yeast extract, and 10 g/liter of sodium chloride (hereinafter also referred to as an "LB medium") was added thereto, and the culturing was continued under constant shaking at 37°C for one hour. The culture medium was spread over an agar plate of ampicillin containing LB medium at 37°C and the culturing was continued overnight at 37°C.

Example 7: Screening of Clone of Leukocidin of MRSA

An ampicillin containing LB agar plate medium was inoculated with the transformed bacteria obtained in Example 6 and the culturing was carried out at 37°C overnight. The agar plate was cooled at 4°C for 30 minutes, and then a Colony/Plaque Screen NEF-978X (DuPont) was laid thereon and allowed to stand at room temperature for about 3 minutes. The screen carrying the adhered bacterial cells was removed from the agar plate and immersed in 0.5N NaOH (750 μl) at room temperature twice (for 2 minutes, respectively). Thereafter, the same procedure was repeated twice, using a 1M Tris buffer (pH 7.5). Then, the DNA-blotted screen was dried at room temperature for 30 minutes. The dried Colony/Plaque Screen was placed in a plastic bag, then 5 ml of a hybridization buffer [0.2% polyvinylpyrrolidone (molecular weight of 40,000), 0.2% Ficoll (molecular weight of 40,000), 0.2% of bovine serum albumin, 0.05M Tris HCl buffer (pH 7.5), 1M sodium chloride, 0.1% sodium phosphate, 1.0% sodium lauryl sulfate (molecular weight of 500,000), and denatured salmon sperm DNA (100 μg/ml)] was added. The bag was sealed and allowed to stand at 65°C overnight, then 100 μl of a solution of the probes (2ng DNA) prepared in Example 3 and labeled by $^{32}$P was added and the whole was allowed to stand at 65°C overnight. The treated Colony/Plaque Screen was washed twice by immersing with agitation in 0.1 x SSC at room temperature for 5 minutes and dried at room temperature for one hour. After hybridizing with the MRSA-DNA probes, washing and drying, the Colony/Plaque Screen was placed in an exposing-cassette and fixed therein. Then, the film (Kodak-X-OMAT-AR XAR-5) was exposed either at - 70°C or room temperature, and developed to determine the transformed colonies. As a result, of the 1000 strains of the examined bacteria, the six strains of No. 11 to No. 16 were selected which hybridized with the probes of the above formulas (3) to (5) and five strains of No. 21 to No. 25 were selected which hybridized with the probes of the above formulas (6) and (7).

Example 8: Size of MRSA-DNAs in Transformed Colonies

To determine the size of the MRSA-DNAs incorporated into the colonies selected by colony hybridization, 25 ml of LB medium containing 50 μg/ml of ampicillin was inoculated with a platinum loopful amount of 11 strains of No. 11 to No. 16 and No. 21 to No. 25, respectively. The medium was cultured while continuously shaking overnight at 37°C, then 1 ml of the resulting cultured medium was withdrawn and transferred to 200 ml of a fresh medium same as above. The medium was cultured until the turbidity thereof at 600 nm reached to 0.4. The cells were harvested by centrifugation and suspended in 4 ml of a 25 mM Tris buffer (pH 8.0) containing 50 mM glucose and 10 mM EDTA. To the suspension, lysozyme was added so that the concentration thereof became 7.5 mg/ml, and the treatment was effected at about 0°C. After 30 minutes, 8 ml of 0.2N NaOH containing 1% sodium laurylsulfate was added to the reaction solution, and the whole was maintained at about 0°C for further 10 minutes. Finally, 5M potassium acetate was added. The mixture was allowed to stand at about 0°C for 10 minutes, and then centrifuged at 19,000 rpm for 30 minutes by a Beckman JA20 centrifuge. The resulting supernatant was mixed with 0.6 volume of isopropanol and the precipitate was collected. To the precipitate, 30 ml of 75% ethyl alcohol was added. The resulting mixture was further centrifuged under the same conditions. The precipitates were collected and dried under vacuum for 5 minutes. The dried precipitates were dissolved in the Tris buffer 3 (2.48 ml), and 2.91 g of cesium chloride and 0.43 ml of ethidium bromide (5 mg/ml) were added thereto. The mixture was centrifuged overnight at 65,000 rpm by a Beckman ALA103.3 centrifuge and the lower band containing a localized circular DNA was taken. The collected component was first treated with 1-butanol to remove the ethidium bromide, and then with ethyl alcohol to precipitate the plasmid DNA. The resulting precipitates were dissolved in the Tris buffer 3.

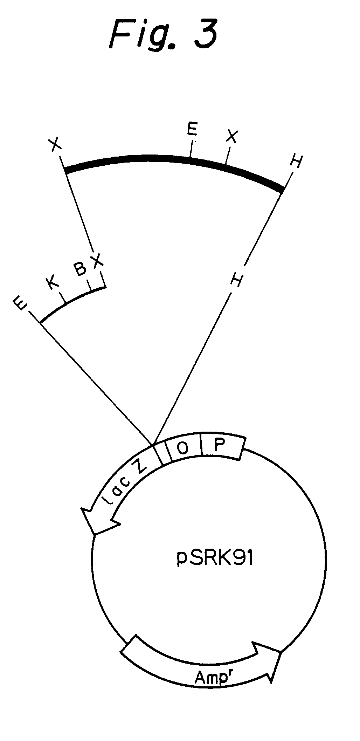

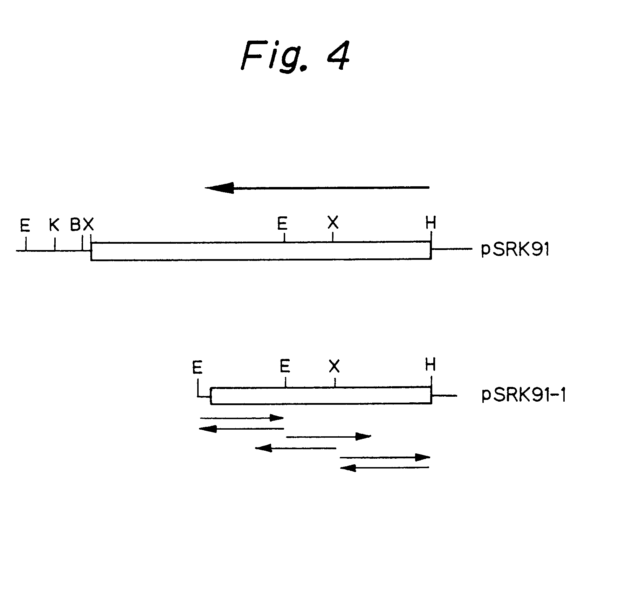

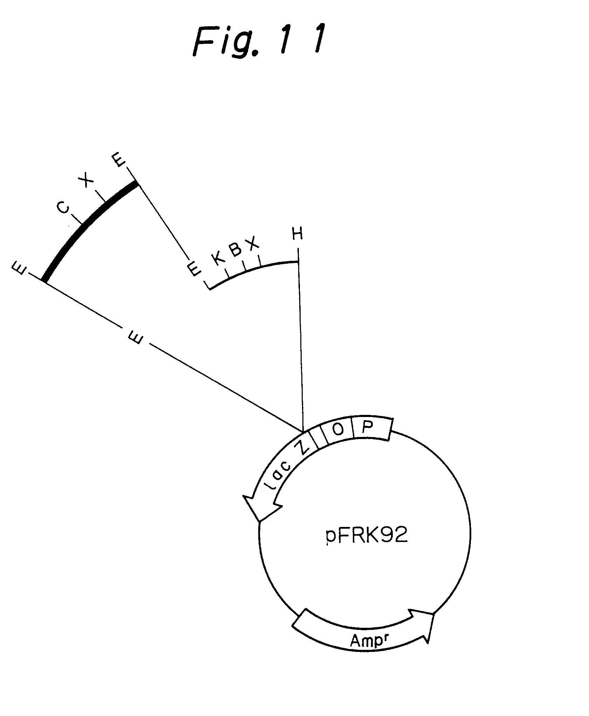

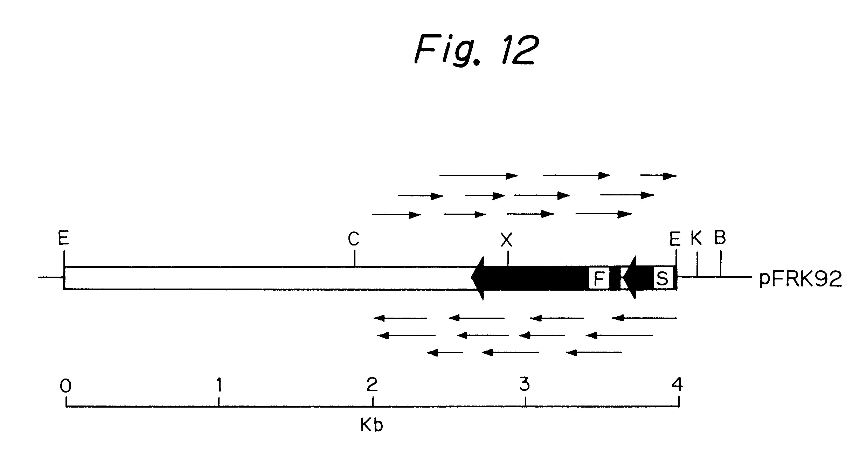

The purified plasmid DNA was digested with XbaI and HindIII in the case of the S-component and with EcoRI in the case of the F-component under the conditions described in Example 5. Thereafter, electrophoresis was carried out for 1.5 hours in a TEA buffer having a composition of 0.8% agarose (Funakoshi, GA-001), 4.4 g/liter of Tris HC1, 1.142 g/liter of glacial acetic acid, and 2 ml/liter of 0.5M EDTA. After the electrophoresis was completed, the agarose gel was immersed in 1.5M saline solution containing 0.5N NaOH at room temperature for 45 minutes while agitating to denature the DNA. Then, the agarose gel was neutralized by immersing in a Tris buffer (pH 8.0) containing 1.5M sodium chloride for 45 minutes at room temperature while agitating. On the agarose gel carrying the denatured DNA, a Gene Screen Plus NEF-976 (DuPont) was laid. Capillary blotting was performed overnight and hybridization was performed as in Example 7. From the results of Southern hybridization, it was found that the sizes of the MRSA-DNAs incorporated into the transformed bacteria No. 11 and No. 21 were about 2.2 kb and about 4 kb, respectively. The plasmid containing the inserted gene DNA encoding leukocidin (S-component) was designated as pSRK91, while the plasmid containing the inserted gene DNA encoding leukocidin (F-component) was designated as pFRK92. The structures of the plasmid pSRK91 and plasmid pFRK92 are shown in Figs. 3 and 11, respectively. The restriction maps of the plasmid pSRK91 and plasmid pFRK92 are shown in Figs. 4 and 12, respectively. In Fig. 4, the arrow above the pSRK91 illustrates the location where the gene encoding leukocidin S-component exists. The forefront end of the arrow corresponds to the C terminus of the S-component protein. Further, pSRK91-1 is a plasmid derived from pSRK91. The arrows under pSRK91-1 show the sequencing strategy. In the pFRK92 of Fig. 12, the thick arrow F shows the location where the gene encoding leukocidin F-component exists. The forefront end of the arrow F corresponds to the C terminus of the F component protein. Further, many thin arrows show the sequencing strategy. Furthermore, the base sequences were determined by the dideoxy method using a DYE primer. The base sequences and the amino acid sequences deduced therefrom are shown in Table 1 and Fig. 5 and in Table 2 and Fig. 13, respectively. In Fig. 5, the amino acid sequence is shown by single-letter notation, while in Fig. 13, the amino acid sequence is shown by three-letter notation.

Table 1

```
GAAGCTTAAA TAAATATTCT CAAGTTAATA AATAATTAAC TTTTAGATGG
ATTCATCAAA AATCGTAAAA AGAACAATTT GTATTTTACA AACATTAATT
AAAAATAAAA GCAAGACATT CGTGCAATCG GTTACCTTAA ATTGTTTACA
ACTGTCAACA ATACCAAGGT TTTATTAACT ATATTTCTCA CAAAATTAGC
TTTTAGCATT CCAAACAAAA AAGGTTAAAT TGAACGGAAT TATGGCATTT
TTAACTTAAT TGTAAAAAAA GTTGATAATG GTCAATTGTT AATGAACAGT
TAATTATAAT AACGTCCAAA ATATATTATT ATTTAATTAA GTTAAATAAA
ATTATAGAAA GACAGTGAAA CTT
```

```
ATG CTT AAA AAT AAA ATA TTA GCT ACA ACT TTA TCT GTG AGC TTA CTT
Met Leu Lys Asn Lys Ile Leu Ala Thr Thr Leu Ser Val Ser Leu Leu
            -25              -20              -15

GCC CCT CTT GCC AAT CCG TTA TTA GAA AAT GCT AAA GCT GCC AAC GAT
Ala Pro Leu Ala Asn Pro Leu Leu Glu Asn Ala Lys Ala Ala Asn Asp
        -10               -5             -1   1

ACT GAA GAC ATC GGT AAA GGA AGC GAT ATA GAA ATT ATC AAA AGG ACA
Thr Glu Asp Ile Gly Lys Gly Ser Asp Ile Glu Ile Ile Lys Arg Thr
    5                 10                 15
```

14

```
GAA GAT AAA ACA AGT AAT AAA TGG GGC GTG ACT CAA AAT ATT CAA TTT
Glu Asp Lys Thr Ser Asn Lys Trp Gly Val Thr Gln Asn Ile Gln Phe
 20                      25                  30                  35

GAT TTT GTG AAG GAT ACA AAA TAT AAC AAA GAT GCT CTG ATA TTA AAG
Asp Phe Val Lys Asp Thr Lys Tyr Asn Lys Asp Ala Leu Ile Leu Lys
             40                  45                  50

ATG CAA GGA TTC ATT AGC TCT AGA ACA ACA TAT TAC AAC TAT AAA AAA
Met Gln Gly Phe Ile Ser Ser Arg Thr Thr Tyr Tyr Asn Tyr Lys Lys
                 55                  60                  65

ACT AAT CAT GTT AAA GCT ATG CGA TGG CCA TTC CAA TAT AAT ATT GGT
Thr Asn His Val Lys Ala Met Arg Trp Pro Phe Gln Tyr Asn Ile Gly
             70                  75                  80

TTA AAA ACA AAT GAT AAA TAT GTT TCT TTA ATT AAT TAT TTA CCT AAA
Leu Lys Thr Asn Asp Lys Tyr Val Ser Leu Ile Asn Tyr Leu Pro Lys
         85                  90                  95

AAT AAA ATT GAA TCT ACA AAC GTG AGT CAG ACA TTA GGA TAC AAT ATC
Asn Lys Ile Glu Ser Thr Asn Val Ser Gln Thr Leu Gly Tyr Asn Ile
100                 105                 110                 115

GGT GGT AAT TTC CAA TCA GCC CCA TCA CTC GGT GGT AAT GGA TCA TTT
Gly Gly Asn Phe Gln Ser Ala Pro Ser Leu Gly Gly Asn Gly Ser Phe
                 120                 125                 130

AAC TAT TCT AAA TCG ATT AGC TAT ACA CAA CAA AAT TAT GTA AGT GAA
Asn Tyr Ser Lys Ser Ile Ser Tyr Thr Gln Gln Asn Tyr Val Ser Glu
             135                 140                 145

GTA GAA CAA CAA AAC TCA AAA AGT GTT TTA TGG GGC GTC AAA GCG AAT
Val Glu Gln Gln Asn Ser Lys Ser Val Leu Trp Gly Val Lys Ala Asn
         150                 155                 160

TCA TTC GCC ACT GAA TCA GGT CAA AAA TCA GCA TTT GAT AGC GAT TTA
Ser Phe Ala Thr Glu Ser Gly Gln Lys Ser Ala Phe Asp Ser Asp Leu
         165                 170                 175

TTT GTA GGC TAC AAA CCT CAT AGT AAA GAT CCT AGA GAT TAT TTC GTT
Phe Val Gly Tyr Lys Pro His Ser Lys Asp Pro Arg Asp Tyr Phe Val
180                 185                 190                 195

CCA GAC AGT GAG TTA CCT CCT CTT GTA CAA AGT GGA TTT AAC CCT TCA
Pro Asp Ser Glu Leu Pro Pro Leu Val Gln Ser Gly Phe Asn Pro Ser
                 200                 205                 210

TTT ATC GCC ACA GTA TCT CAT GAA AAA GGT TCA AGC GAT ACA AGC GAA
Phe Ile Ala Thr Val Ser His Glu Lys Gly Ser Ser Asp Thr Ser Glu
             215                 220                 225

TTT GAA ATT ACT TAC GGA AGA AAC ATG GAT GTC ACT CAT GCC ATT AAA
Phe Glu Ile Thr Tyr Gly Arg Asn Met Asp Val Thr His Ala Ile Lys
         230                 235                 240

AGA TCA ACG CAT TAT GGC AAC AGT TAT TTA GAC GGA CAT AGA GTC CAT
Arg Ser Thr His Tyr Gly Asn Ser Tyr Leu Asp Gly His Arg Val His
         245                 250                 255

AAT GCA TTC GTA AAT AGA AAC TAT ACT GTT AAA TAC GAC GTC AAT TGG
Asn Ala Phe Val Asn Arg Asn Tyr Thr Val Lys Tyr Glu Val Asn Trp
260                 265                 270                 275
```

```
AAG ACT CAT GAA ATC AAG GTG AAA GGA CAG AAT
Lys Thr His Glu Ile Lys Val Lys Gly Gln Asn
                280                    285
```

```
TGATATCAAA ATGAATAAAT TAGTCAAATC ATCCGTTGCT ACATCTATGG
CATTATTATT ACTTTCTGGT ACTGCTAATG CTGAGGTAAA ATAACACCAG
TCAGCGTAAA AAAGTCGATG ACAAGGTTAC TTTATACAAA CCACCAGC
```

Table 2

```
GAATTCATTC GCCACTGAAT CAGGTCAAAA ATCAGCATTT GATAGCGATT
TATTTGTAGG CTACAAACCT CATAGTAAAG ATCCTAGAGA TTATTTCGTT
CAAGACAGTG AGTTACCTCC TCTTGTACAA AGTGGATTTA ACCCTTCATT
TATCGCCACA GTATCTCATG AAAAAGGTTC AAGCGATACA AGCGAATTTG
AAATTACTTA CGGAAGAAAC ATGGATGTCA CTCATGCCAT TAAAAGATCA
ACGCATTATG GCAACAGTTA TTTAGACGGA CATAGAGTCC ATAATGCATT
CGTAAATAGA AACTATACTG TTAAATACGA GGTCAATTGG AAGACTCATG
AAATCAAGGT GAAAGGACAG AATTGAT
```

```
ATG AAA ATG AAT AAA TTA GTC AAA TCA TCC GTT GCT ACA TCT ATG GCA
Met Lys Met Asn Lys Leu Val Lys Ser Ser Val Ala Thr Ser Met Ala
-25                 -20             -15                     -10
```

```
TTA TTA TTA CTT TCT GGT ACT GCT AAT GCT GAA GGT AAA ATA ACA CCA
Leu Leu Leu Leu Ser Gly Thr Ala Asn Ala Glu Gly Lys Ile Thr Pro
             -5             -1  +1              5
```

```
GTC AGC GTA AAA AAA GTC GAT GAC AAA GTT ACT TTA TAC AAA ACA ACA
Val Ser Val Lys Lys Val Asp Asp Lys Val Thr Leu Tyr Lys Thr Thr
         10              15                  20
```

```
GCC ACA GCA GAT TCT GAT AAG TTT AAA ATT TCA CAG ATT TTA ACA TTT
Ala Thr Ala Asp Ser Asp Lys Phe Lys Ile Ser Gln Ile Leu Thr Phe
         25              30                  35
```

```
AAT TTC ATC AAA GAT AAA AGT TAT GAT AAA GAT ACT TTA GTA CTT AAA
Asn Phe Ile Lys Asp Lys Ser Tyr Asp Lys Asp Thr Leu Val Leu Lys
 40                  45                  50                      55
```

```
GCT ACT GGG AAT ATT AAC TCA GGC TTT GTG AAA CCT AAT CCT AAT GAC
Ala Thr Gly Asn Ile Asn Ser Gly Phe Val Lys Pro Asn Pro Asn Asp
                 60              65                  70
```

```
TAT GAC TTT TCA AAA TTA TAT TGG GGA GCT AAA TAC AAT GTA TCT ATA
Tyr Asp Phe Ser Lys Leu Tyr Trp Gly Ala Lys Tyr Asn Val Ser Ile
             75              80                  85
```

```
AGC TCA CAA TCT AAT GAT TCA GTA AAC GCT GTT GAT TAT GCA CCA AAA
Ser Ser Gln Ser Asn Asp Ser Val Asn Ala Val Asp Tyr Ala Pro Lys
         90              95                  100
```

```
AAT CAA AAT GAA GAG TTT CAA GTT CAA AAT ACT TTA GGC TAT ACA TTT
Asn Gln Asn Glu Glu Phe Gln Val Gln Asn Thr Leu Gly Tyr Thr Phe
         105                 110                 115
```

```
GGT GGT GAC ATT AGT ATC TCT AAT GGT TTA TCT GGT GGA CTT AAT GGA
Gly Gly Asp Ile Ser Ile Ser Asn Gly Leu Ser Gly Gly Leu Asn Gly
120                 125                 130                     135
```

```
AAT ACA GCT TTT TCT GAA ACA ATT AAT TAT AAA CAA GAA AGT TAC AGA
Asn Thr Ala Phe Ser Glu Thr Ile Asn Tyr Lys Gln Glu Ser Tyr Arg
                 140                 145                 150
```

16

```
ACA TTA AGT CGC AAC ACA AAT TAT AAA AAT GTT GGC TGG GGA GTT GAA
Thr Leu Ser Arg Asn Thr Asn Tyr Lys Asn Val Gly Trp Gly Val Glu
            155             160             165

GCA CAT AAA ATT ATG AAT GGT TGG GGA CCT TAT GGA AGA GAT AGC TTC
Ala His Lys Ile Met Asn Gly Trp Gly Pro Tyr Gly Arg Asp Ser Phe
            170             175             180

CAC CCA ACA TAT GGT AAT GAA CTC TTC TTA GCT GGC AGA CAA AGC AGT
His Pro Thr Tyr Gly Asn Glu Leu Phe Leu Ala Gly Arg Gln Ser Ser
            185             190             195

GCA TAC GCT GGC CAA AAC TTC ATA GCG CAA CAC CAA ATG CCA TTA TTA
Ala Tyr Ala Gly Gln Asn Phe Ile Ala Gln His Gln Met Pro Leu Leu
200             205             210             215

TCT AGA AGT AAC TTC AAT CCC AGA TTT TTA AGC GTA CTA TCA CAC AGA
Ser Arg Ser Asn Phe Asn Pro Arg Phe Leu Ser Val Leu Ser His Arg
            220             225             230

CAA GAT GCC GCT AAA AAA TCT AAA ATT ACA GTA ACT TAT CAA CGT GAA
Gln Asp Ala Ala Lys Lys Ser Lys Ile Thr Val Thr Tyr Gln Arg Glu
            235             240             245

ATG GAT TTA TAC CAA ATT CGT TGG AAT GGC TTC TAC TGG GCA GGC GCA
Met Asp Leu Tyr Gln Ile Arg Trp Asn Gly Phe Tyr Trp Ala Gly Ala
            250             255             260

AAT TAT AAA AAC TTT AAA ACT AGA ACA TTT AAA TCA ACA TAT GAA ATT
Asn Tyr Lys Asn Phe Lys Thr Arg Thr Phe Lys Ser Thr Tyr Glu Ile
            265             270             275

GAT TGG GAA AAT CAC AAA GTG AAA TTG TTA GAT ACA AAA GAA ACT GAA
Asp Trp Glu Asn His Lys Val Lys Leu Leu Asp Thr Lys Glu Thr Glu
280             285             290             295

AAC AAT AAA
Asn Asn Lys
        298

TAGCTAGTAA AACACGGTCG CCAACAGTAA TTGTGACGAC CGTGTTTTGA
TTTATTATCT TAGTAAGACT GCCATTCTTT TTCTCAATGT GAGATATAAA
GGAATAGCTA CAATTAAAGT GAATATTACG CCTGGAATCG CGTTTAACAA
CACTACCCAC ACAGGTAAAT TTAAAATAAT AGATAATAGT AGCCTAGATA
CCAAACTGCC TAATACACTT GATAAAACTA ATGATAGTAC ATTTATTTTC
AATAAATAAA CAACTGCAAT AGCTATAACT CTAAATATAA TAGAAATAAT
CACATTAATT GGATTAAATA CGCCAAATAT CAGTAATAAT ACGCTTGATA
AAAGCCCACC TAAAAAGTAC TTTTTAATTC CAAAGAAAGC TAATATCAAT
AATGCTGCCG GTGCAGATAA TTGAAAATCT AATCCTGGTA TAATGGACGG
TATTTTCAAA ACTGCCAAAA TGGTTAAAAT CGCAGCCATG ACACTAATTT
GAGTAATATC TTTTGATGTC ATACTAAAAC CCCTATACCG TTTCATAAAC
TTCTTGCTTC GGTGTGCTTT CTAAAAATGA TATGTAATGA TTTAAATCAA
TACAATCGTC CACAAATATT ATTCTGCCTC CATATCTCGT ATTAACTGGT
TTAATATCAA ATAATC
```

## Example 9

(a) Identification of Translation Product *In Vitro* Encoded by Plasmids pSRK91 and pFRK92

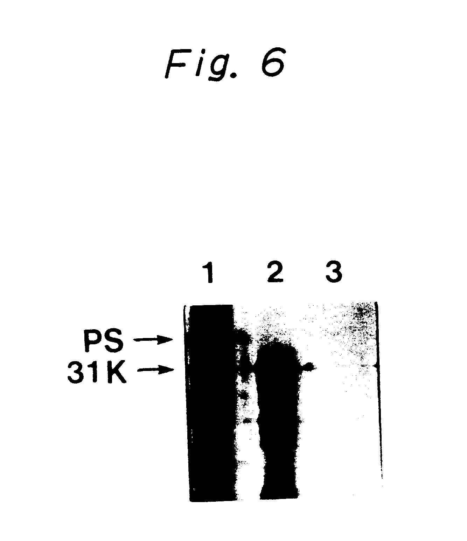

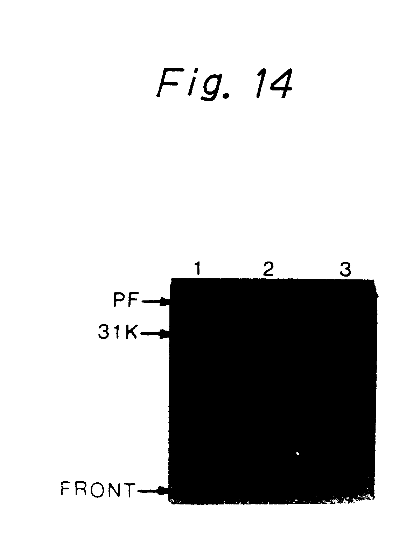

The products *in vitro* from the plasmid pSRK91 (transformed bacteria No. 11 of Example 7) and the plasmid pFRK92 (transformed bacteria No. 21 of Example 7) were confirmed by the transcription/translation method [Zuday, *Ann Rev. Genet*. 7, 267-287 (1974)]. That is, the products *in vitro* labeled with ($^{35}$S) methionine were analyzed by electrophoresis on an SDS-PAGE and autoradiography. The results are shown in Figs. 6 and 14. An about 35 kd polypeptide (PS) was observed in the pSRK91, and an about 37 kd polypeptide (PF) was observed in the pFRK92. Such proteins were not observed when pUC119 was similarly treated. In view of the results obtained in Example 1 that the molecular weight of the S-component was about 32 kd, while the molecular weight of the F-component was about 34 kd, it is possible to assume from the above results in this Example that the approximately 35 kd polypeptide (PS) and approximately 37 kd polypeptide (PF) are the prematured S-protein (preproleukocidin S) and the prematured F-protein (preproleukocidin F), respectively.

(b) Identification of S- and F-Components *in Vivo*

(b-1) Preparation of Antibody

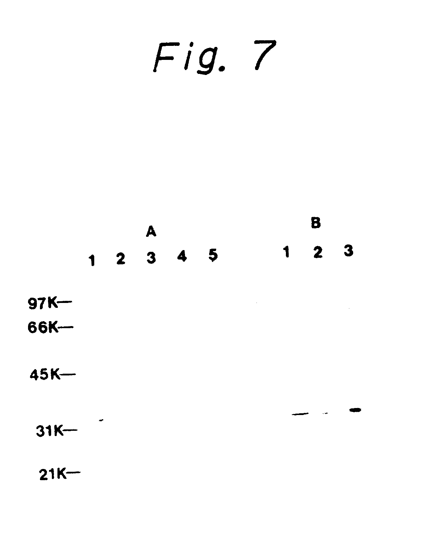

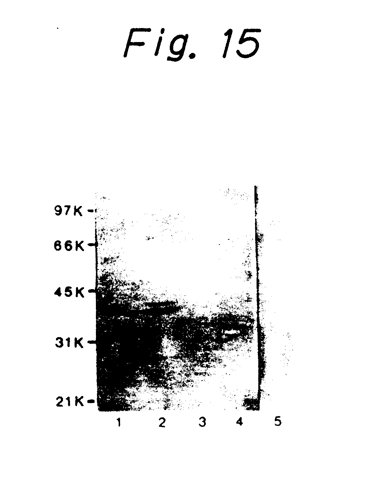

A rabbit was immunized with the leukocidin (prepared in Example 1) as the antigen by the usual method to prepare antiserum. Solid ammonium sulfate was added to the resulting antiserum to 50% saturation. The precipitate was collected by centrifuging, dissolved in PBS, and fractionated by adding ammonium sulfate to 33% saturation, and centrifuged to obtain the precipitate. The precipitate was dissolved in 17.5 mM phosphate buffer (pH 6.8). The solution was dialyzed against the same buffer, then purified by a DEAE cellulose column. The resulting IgG component was used as the antibody. (b-2) The transformed bacteria No. 11 and No. 21 of Example 7 were cultured in the presence of IPTG (2 mM) in an LB medium containing ampicillin (5 x $10^8$ cells/ml: mid Log phase). The cells were harvested by centrifugation, washed once with a 10 mM Tris-HCl buffer (pH 7.5), treated with ultrasonic waves, and then centrifuged (15,000 x g, 10 minutes). The cell extract was partially purified under the conditions of Example 1. The above cultured medium and the partially purified product of the cell extract were analyzed by electrophoresis on an SDS-PAGE and then the Western blotting using the above antibody. As the secondary antibody, an antirabbit antibody labeled with alkali phosphatase was used in the detection. The results are shown in Fig. 7(A) and 15. The results of a test without IPTG are shown in Fig. 7(B).

As above, the matured types of the molecular weights of 32 kd and 34 kd were not observed in the culture medium, but observed in the cell extract prepared by ultrasonic treatment. Further, the matured types were observed only in DH5$\alpha$ (pSRK91) and DH5$\alpha$ (pFRK92), but were not observed either in the culture medium and cell extract from DH5$\alpha$ (pUC119). In DH5$\alpha$ (pSRK91), the 32 kd matured type (proleukocidin S) was observed in the cell extract, even in the absence of the PPTG. The same result was observed in DH5$\alpha$ (pFRK92).

(c) Localization of Expressed S- and F-Components in DH5$\alpha$ (pSRK91) and DH5$\alpha$ (pFRK92)

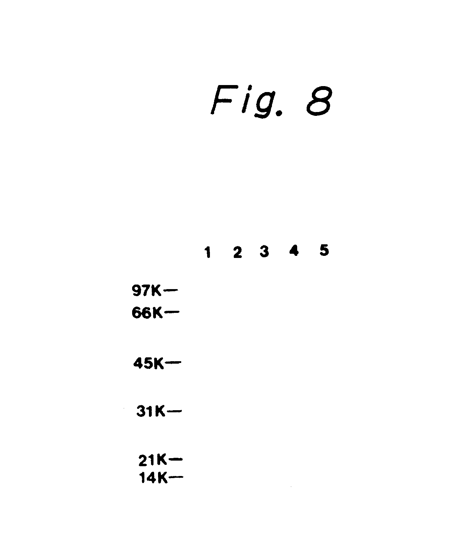

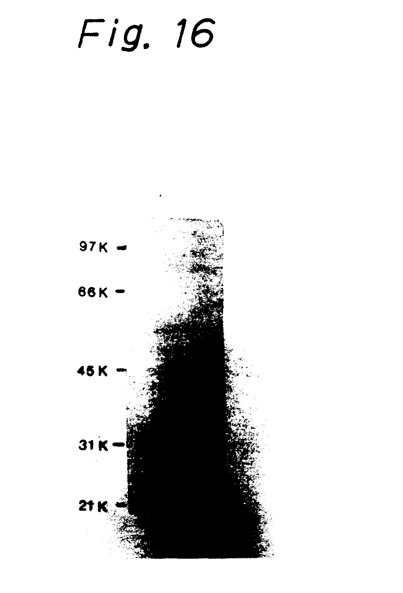

DH5$\alpha$ (pSRK91) and DH5$\alpha$ (pFRK92) were cultured at 37°C in an LB medium containing ampicillin (100 $\mu$g/ml) (mid-exponential phase). The culture medium was washed once with 10 mM Tris HCL buffer (pH 7.5) and shocked by the method of Heppel [*Science,* 156, 1451-1455 (1967)] to obtain a periplasmic fraction (space between outer and inner membranes) and a shocked cell fraction. The fractions were analyzed by western blotting in the same manner as in (b-2). The results are shown in Figs. 8 and 16. The approximately 32 kb proleukocidin S was found in the periplasmic fraction (between outer membrane and inner membrane) of the DH5$\alpha$ (pSRK91) and the approximately 34 kb proleukocidin F was found in the periplasmic fraction (between outer membrane and inner membrane) of the DH5$\alpha$ (pFRK92).

From the above results, it was found that the preproleukocidin S (35 kd) and proleukocidin S (32 kd), and preproleukocidin F (37 kd) and proleukocidin F (34 kd) obtained from the transformed bacteria can be used as antigens for preparation of an antileukocidin F antibody and may be used in the diagnosis of MRSA.

Example 10: Evaluation of Leukocidin as a Marker for MRSA Detection

Twenty one strains of MRSA (strains isolated from patients, School of Medicine, Shinshu University) was cultured under the conditions as in Example 1. The partially purified proleukocidin component was examined by Western blotting under the conditions described in Example 9. As a result, it was found that all of the specimens used in the test produced leukocidin (S and F). However, normal *Staphylococcus aureus* was tested in the same manner, whereupon no specimen producing leukocidin was found.

Hitherto, detection of MRSA was time-consuming and troublesome, because there was no marker specific to MRSA and procedures such as isolation of the bacteria were necessary. However, the present invention revealed that MRSA specifically produces leukocidin (S- and F-components) and proleukocidin (S-and F-components), and these proteins can be used as markers for identification of MRSA. Further, in the present invention, the entire structures of leukocidin (S- and F-components) and proleukocidin (S- and F-components) were determined for the first time, and the antigen necessary for preparation of an antibody used for immunodiagnosis of MRSA can be produced, using gene manipulation technique. As a result, the present invention is effective to find MRSA infected patients in an early stage and can contribute to prevent the nosocomial infection of MRSA in question. Further, the elucidation of the base sequences encoding leukocidin and proleukocidin made it possible to definitely diagnose MRSA.

**Claims**

1.  A method for detecting methicillin resistant *Staphylococcus aureus* characterized in that an antibody against proleukocidin or leukocidin is used.

2.  A peptide having an amino acid sequence of the formula:

```
Ala Asn Asp Thr Glu Asp Ile Gly Lys Gly Ser Asp Ile Glu Ile Ile
Lys Arg Thr Glu Asp Lys Thr Ser Asn Lys Trp Gly Val Thr Gln Asn
Ile Gln Phe Asp Phe Val Lys Asp Thr Lys Tyr Asn Lys Asp Ala Leu
Ile Leu Lys Met Gln Gly Phe Ile Ser Ser Arg Thr Thr Tyr Tyr Asn
Tyr Lys Lys Thr Asn His Val Lys Ala Met Arg Trp Pro Phe Gln Tyr
Asn Ile Gly Leu Lys Thr Asn Asp Lys Tyr Val Ser Leu Ile Asn Tyr
Leu Pro Lys Asn Lys Ile Glu Ser Thr Asn Val Ser Gln Thr Leu Gly
Tyr Asn Ile Gly Gly Asn Phe Gln Ser Ala Pro Ser Leu Gly Gly Asn
Gly Ser Phe Asn Tyr Ser Lys Ser Ile Ser Tyr Thr Gln Gln Asn Tyr
Val Ser Glu Val Glu Gln Gln Asn Ser Lys Ser Val Leu Trp Gly Val
Lys Ala Asn Ser Phe Ala Thr Glu Ser Gly Gln Lys Ser Ala Phe Asp
Ser Asp Leu Phe Val Gly Tyr Lys Pro His Ser Lys Asp Pro Arg Asp
Tyr Phe Val Pro Asp Ser Glu Leu Pro Pro Leu Val Gln Ser Gly Phe
Asn Pro Ser Phe Ile Ala Thr Val Ser His Glu Lys Gly Ser Ser Asp
Thr Ser Glu Phe Glu Ile Thr Tyr Gly Arg Asn Met Asp Val Thr His
Ala Ile Lys Arg Ser Thr His Tyr Gly Asn Ser Tyr Leu Asp Gly His
Arg Val His Asn Ala Phe Val Asn Arg Asn Tyr Thr Val Lys Tyr Glu
Val Asn Trp Lys Thr His Glu Ile Lys Val Lys Gly Gln Asn
```

3.  A peptide having an amino acid sequence of the formula:

Met Leu Lys Asn Lys Ile Leu Ala Thr Thr Leu Ser Val Ser Leu Leu
Ala Pro Leu Ala Asn Pro Leu Leu Glu Asn Ala Lys Ala Ala Asn Asp
Thr Glu Asp Ile Gly Lys Gly Ser Asp Ile Glu Ile Ile Lys Arg Thr
Glu Asp Lys Thr Ser Asn Lys Trp Gly Val Thr Gln Asn Ile Gln Phe
Asp Phe Val Lys Asp Thr Lys Tyr Asn Lys Asp Ala Leu Ile Leu Lys
Met Gln Gly Phe Ile Ser Ser Arg Thr Thr Tyr Tyr Asn Tyr Lys Lys
Thr Asn His Val Lys Ala Met Arg Trp Pro Phe Gln Tyr Asn Ile Gly
Leu Lys Thr Asn Asp Lys Tyr Val Ser Leu Ile Asn Tyr Leu Pro Lys
Asn Lys Ile Glu Ser Thr Asn Val Ser Gln Thr Leu Gly Tyr Asn Ile
Gly Gly Asn Phe Gln Ser Ala Pro Ser Leu Gly Gly Asn Gly Ser Phe
Asn Tyr Ser Lys Ser Ile Ser Tyr Thr Gln Gln Asn Tyr Val Ser Glu
Val Glu Gln Gln Asn Ser Lys Ser Val Leu Trp Gly Val Lys Ala Asn
Ser Phe Ala Thr Glu Ser Gly Gln Lys Ser Ala Phe Asp Ser Asp Leu
Phe Val Gly Tyr Lys Pro His Ser Lys Asp Pro Arg Asp Tyr Phe Val

Pro Asp Ser Glu Leu Pro Pro Leu Val Gln Ser Gly Phe Asn Pro Ser
Phe Ile Ala Thr Val Ser His Glu Lys Gly Ser Ser Asp Thr Ser Glu
Phe Glu Ile Thr Tyr Gly Arg Asn Met Asp Val Thr His Ala Ile Lys
Arg Ser Thr His Tyr Gly Asn Ser Tyr Leu Asp Gly His Arg Val His
Asn Ala Phe Val Asn Arg Asn Tyr Thr Val Lys Tyr Glu Val Asn Trp
Lys Thr His Glu Ile Lys Val Lys Gly Gln Asn

4. A DNA containing a base sequence encoding a peptide having an amino acid sequence of the formula:

```
Ala Asn Asp Thr Glu Asp Ile Gly Lys Gly Ser Asp Ile Glu Ile Ile
Lys Arg Thr Glu Asp Lys Thr Ser Asn Lys Trp Gly Val Thr Gln Asn
Ile Gln Phe Asp Phe Val Lys Asp Thr Lys Tyr Asn Lys Asp Ala Leu
Ile Leu Lys Met Gln Gly Phe Ile Ser Ser Arg Thr Thr Tyr Tyr Asn
Tyr Lys Lys Thr Asn His Val Lys Ala Met Arg Trp Pro Phe Gln Tyr
Asn Ile Gly Leu Lys Thr Asn Asp Lys Tyr Val Ser Leu Ile Asn Tyr
Leu Pro Lys Asn Lys Ile Glu Ser Thr Asn Val Ser Gln Thr Leu Gly
Tyr Asn Ile Gly Gly Asn Phe Gln Ser Ala Pro Ser Leu Gly Gly Asn
Gly Ser Phe Asn Tyr Ser Lys Ser Ile Ser Tyr Thr Gln Gln Asn Tyr
Val Ser Glu Val Glu Gln Gln Asn Ser Lys Ser Val Leu Trp Gly Val
Lys Ala Asn Ser Phe Ala Thr Glu Ser Gly Gln Lys Ser Ala Phe Asp
Ser Asp Leu Phe Val Gly Tyr Lys Pro His Ser Lys Asp Pro Arg Asp
Tyr Phe Val Pro Asp Ser Glu Leu Pro Pro Leu Val Gln Ser Gly Phe
Asn Pro Ser Phe Ile Ala Thr Val Ser His Glu Lys Gly Ser Ser Asp
Thr Ser Glu Phe Glu Ile Thr Tyr Gly Arg Asn Met Asp Val Thr His
Ala Ile Lys Arg Ser Thr His Tyr Gly Asn Ser Tyr Leu Asp Gly His
Arg Val His Asn Ala Phe Val Asn Arg Asn Tyr Thr Val Lys Tyr Glu
Val Asn Trp Lys Thr His Glu Ile Lys Val Lys Gly Gln Asn
```

**5.** A DNA containing a base sequence encoding a peptide having an amino acid sequence of the formula:

```
Met Leu Lys Asn Lys Ile Leu Ala Thr Thr Leu Ser Val Ser Leu Leu
Ala Pro Leu Ala Asn Pro Leu Leu Glu Asn Ala Lys Ala Ala Asn Asp
Thr Glu Asp Ile Gly Lys Gly Ser Asp Ile Glu Ile Ile Lys Arg Thr
Glu Asp Lys Thr Ser Asn Lys Trp Gly Val Thr Gln Asn Ile Gln Phe
Asp Phe Val Lys Asp Thr Lys Tyr Asn Lys Asp Ala Leu Ile Leu Lys
Met Gln Gly Phe Ile Ser Ser Arg Thr Thr Tyr Tyr Asn Tyr Lys Lys
Thr Asn His Val Lys Ala Met Arg Trp Pro Phe Gln Tyr Asn Ile Gly
Leu Lys Thr Asn Asp Lys Tyr Val Ser Leu Ile Asn Tyr Leu Pro Lys
Asn Lys Ile Glu Ser Thr Asn Val Ser Gln Thr Leu Gly Tyr Asn Ile
Gly Gly Asn Phe Gln Ser Ala Pro Ser Leu Gly Gly Asn Gly Ser Phe
```

Asn Tyr Ser Lys Ser Ile Ser Tyr Thr Gln Gln Asn Tyr Val Ser Glu
Val Glu Gln Gln Asn Ser Lys Ser Val Leu Trp Gly Val Lys Ala Asn
Ser Phe Ala Thr Glu Ser Gly Gln Lys Ser Ala Phe Asp Ser Asp Leu
Phe Val Gly Tyr Lys Pro His Ser Lys Asp Pro Arg Asp Tyr Phe Val
Pro Asp Ser Glu Leu Pro Pro Leu Val Gln Ser Gly Phe Asn Pro Ser
Phe Ile Ala Thr Val Ser His Glu Lys Gly Ser Ser Asp Thr Ser Glu
Phe Glu Ile Thr Tyr Gly Arg Asn Met Asp Val Thr His Ala Ile Lys
Arg Ser Thr His Tyr Gly Asn Ser Tyr Leu Asp Gly His Arg Val His
Asn Ala Phe Val Asn Arg Asn Tyr Thr Val Lys Tyr Glu Val Asn Trp
Lys Thr His Glu Ile Lys Val Lys Gly Gln Asn

6. A peptide having an amino acid sequence of the formula:

Ala Glu Gly Lys Ile Thr Pro Val Ser Val Lys Lys Val Asp Asp Lys
Val Thr Leu Tyr Lys Thr Thr Ala Thr Ala Asp Ser Asp Lys Phe Lys
Ile Ser Gln Ile Leu Thr Phe Asn Phe Ile Lys Asp Lys Ser Tyr Asp
Lys Asp Thr Leu Val Leu Lys Ala Thr Gly Asn Ile Asn Ser Gly Phe
Val Lys Pro Asn Pro Asn Asp Tyr Asp Phe Ser Lys Leu Tyr Trp Gly
Ala Lys Tyr Asn Val Ser Ile Ser Ser Gln Ser Asn Asp Ser Val Asn
Ala Val Asp Tyr Ala Pro Lys Asn Gln Asn Glu Glu Phe Gln Val Gln
Asn Thr Leu Gly Tyr Thr Phe Gly Gly Asp Ile Ser Ile Ser Asn Gly
Leu Ser Gly Gly Leu Asn Gly Asn Thr Ala Phe Ser Glu Thr Ile Asn
Tyr Lys Gln Glu Ser Tyr Arg Thr Leu Ser Arg Asn Thr Asn Tyr Lys
Asn Val Gly Trp Gly Val Glu Ala His Lys Ile Met Asn Gly Trp Gly
Pro Tyr Gly Arg Asp Ser Phe His Pro Thr Tyr Gly Asn Glu Leu Phe
Leu Ala Gly Arg Gln Ser Ser Ala Tyr Ala Gly Gln Asn Phe Ile Ala
Gln His Gln Met Pro Leu Leu Ser Arg Ser Asn Phe Asn Pro Arg Phe
Leu Ser Val Leu Ser His Arg Gln Asp Ala Ala Lys Lys Ser Lys Ile
Thr Val Thr Tyr Gln Arg Glu Met Asp Leu Tyr Gln Ile Arg Trp Asn
Gly Phe Tyr Trp Ala Gly Ala Asn Tyr Lys Asn Phe Lys Thr Arg Thr
Phe Lys Ser Thr Tyr Glu Ile Asp Trp Glu Asn His Lys Val Lys Leu
Leu Asp Thr Lys Glu Thr Glu Asn Asn Lys

7. A peptide having an amino acid sequence of the formula:

Met Lys Met Asn Lys Leu Val Lys Ser Ser Val Ala Thr Ser Met Ala
Leu Leu Leu Leu Ser Gly Thr Ala Asn Ala Glu Gly Lys Ile Thr Pro
Val Ser Val Lys Lys Val Asp Asp Lys Val Thr Leu Tyr Lys Thr Thr
Ala Thr Ala Asp Ser Asp Lys Phe Lys Ile Ser Gln Ile Leu Thr Phe
Asn Phe Ile Lys Asp Lys Ser Tyr Asp Lys Asp Thr Leu Val Leu Lys
Ala Thr Gly Asn Ile Asn Ser Gly Phe Val Lys Pro Asn Pro Asn Asp
Tyr Asp Phe Ser Lys Leu Tyr Trp Gly Ala Lys Tyr Asn Val Ser Ile

Ser Ser Gln Ser Asn Asp Ser Val Asn Ala Val Asp Tyr Ala Pro Lys
Asn Gln Asn Glu Glu Phe Gln Val Gln Asn Thr Leu Gly Tyr Thr Phe
Gly Gly Asp Ile Ser Ile Ser Asn Gly Leu Ser Gly Gly Leu Asn Gly
Asn Thr Ala Phe Ser Glu Thr Ile Asn Tyr Lys Gln Glu Ser Tyr Arg
Thr Leu Ser Arg Asn Thr Asn Tyr Lys Asn Val Gly Trp Gly Val Glu
Ala His Lys Ile Met Asn Gly Trp Gly Pro Tyr Gly Arg Asp Ser Phe
His Pro Thr Tyr Gly Asn Glu Leu Phe Leu Ala Gly Arg Gln Ser Ser
Ala Tyr Ala Gly Gln Asn Phe Ile Ala Gln His Gln Met Pro Leu Leu
Ser Arg Ser Asn Phe Asn Pro Arg Phe Leu Ser Val Leu Ser His Arg
Gln Asp Ala Ala Lys Lys Ser Lys Ile Thr Val Thr Tyr Gln Arg Glu
Met Asp Leu Tyr Gln Ile Arg Trp Asn Gly Phe Tyr Trp Ala Gly Ala
Asn Tyr Lys Asn Phe Lys Thr Arg Thr Phe Lys Ser Thr Tyr Glu Ile
Asp Trp Glu Asn His Lys Val Lys Leu Leu Asp Thr Lys Glu Thr Glu
Asn Asn Lys

8.  A DNA containing a base sequence encoding a peptide having an amino acid sequence of the formula:

```
Ala Glu Gly Lys Ile Thr Pro Val Ser Val Lys Lys Val Asp Asp Lys
Val Thr Leu Tyr Lys Thr Thr Ala Thr Ala Asp Ser Asp Lys Phe Lys
Ile Ser Gln Ile Leu Thr Phe Asn Phe Ile Lys Asp Lys Ser Tyr Asp
Lys Asp Thr Leu Val Leu Lys Ala Thr Gly Asn Ile Asn Ser Gly Phe
Val Lys Pro Asn Pro Asn Asp Tyr Asp Phe Ser Lys Leu Tyr Trp Gly
Ala Lys Tyr Asn Val Ser Ile Ser Ser Gln Ser Asn Asp Ser Val Asn
Ala Val Asp Tyr Ala Pro Lys Asn Gln Asn Glu Glu Phe Gln Val Gln
Asn Thr Leu Gly Tyr Thr Phe Gly Gly Asp Ile Ser Ile Ser Asn Gly
Leu Ser Gly Gly Leu Asn Gly Asn Thr Ala Phe Ser Glu Thr Ile Asn
Tyr Lys Gln Glu Ser Tyr Arg Thr Leu Ser Arg Asn Thr Asn Tyr Lys
Asn Val Gly Trp Gly Val Glu Ala His Lys Ile Met Asn Gly Trp Gly
Pro Tyr Gly Arg Asp Ser Phe His Pro Thr Tyr Gly Asn Glu Leu Phe
Leu Ala Gly Arg Gln Ser Ser Ala Tyr Ala Gly Gln Asn Phe Ile Ala
Gln His Gln Met Pro Leu Leu Ser Arg Ser Asn Phe Asn Pro Arg Phe
Leu Ser Val Leu Ser His Arg Gln Asp Ala Ala Lys Lys Ser Lys Ile
Thr Val Thr Tyr Gln Arg Glu Met Asp Leu Tyr Gln Ile Arg Trp Asn
Gly Phe Tyr Trp Ala Gly Ala Asn Tyr Lys Asn Phe Lys Thr Arg Thr
Phe Lys Ser Thr Tyr Glu Ile Asp Trp Glu Asn His Lys Val Lys Leu
Leu Asp Thr Lys Glu Thr Glu Asn Asn Lys
```

9.   A DNA containing a base sequence encoding a peptide having an amino acid sequence of the formula:

```
Met Lys Met Asn Lys Leu Val Lys Ser Ser Val Ala Thr Ser Met Ala
```

```
Leu Leu Leu Leu Ser Gly Thr Ala Asn Ala Glu Gly Lys Ile Thr Pro
Val Ser Val Lys Lys Val Asp Asp Lys Val Thr Leu Tyr Lys Thr Thr
Ala Thr Ala Asp Ser Asp Lys Phe Lys Ile Ser Gln Ile Leu Thr Phe
Asn Phe Ile Lys Asp Lys Ser Tyr Asp Lys Asp Thr Leu Val Leu Lys
Ala Thr Gly Asn Ile Asn Ser Gly Phe Val Lys Pro Asn Pro Asn Asp
Tyr Asp Phe Ser Lys Leu Tyr Trp Gly Ala Lys Tyr Asn Val Ser Ile
Ser Ser Gln Ser Asn Asp Ser Val Asn Ala Val Asp Tyr Ala Pro Lys
Asn Gln Asn Glu Glu Phe Gln Val Gln Asn Thr Leu Gly Tyr Thr Phe
Gly Gly Asp Ile Ser Ile Ser Asn Gly Leu Ser Gly Gly Leu Asn Gly
Asn Thr Ala Phe Ser Glu Thr Ile Asn Tyr Lys Gln Glu Ser Tyr Arg
Thr Leu Ser Arg Asn Thr Asn Tyr Lys Asn Val Gly Trp Gly Val Glu
Ala His Lys Ile Met Asn Gly Trp Gly Pro Tyr Gly Arg Asp Ser Phe
His Pro Thr Tyr Gly Asn Glu Leu Phe Leu Ala Gly Arg Gln Ser Ser
Ala Tyr Ala Gly Gln Asn Phe Ile Ala Gln His Gln Met Pro Leu Leu
Ser Arg Ser Asn Phe Asn Pro Arg Phe Leu Ser Val Leu Ser His Arg
Gln Asp Ala Ala Lys Lys Ser Lys Ile Thr Val Thr Tyr Gln Arg Glu
Met Asp Leu Tyr Gln Ile Arg Trp Asn Gly Phe Tyr Trp Ala Gly Ala
Asn Tyr Lys Asn Phe Lys Thr Arg Thr Phe Lys Ser Thr Tyr Glu Ile
Asp Trp Glu Asn His Lys Val Lys Leu Leu Asp Thr Lys Glu Thr Glu
Asn Asn Lys
```

10. A method for preparing DNA encoding leukocidin or proleukocidin characterized by isolating a chromosomal DNA from methicillin resistant *Staphylococcus aureus*, digesting the DNA with a restriction enzyme, inserting the resulting DNA fragment into a vector, transforming a host with the vector to prepare a DNA library, using a synthetic DNA encoding a partial amino acid sequence of leukocidin or proleukocidin purified from the methicillin resistant *Staphylococcus aureus* as a probe to select a DNA encoding proleukocidin or proleukocidin from said DNA library, and cloning the resulting DNA.

11. A method for preparing leukocidin or proleukocidin characterized by ligating a DNA encoding leukocidin or proleukocidin into an expression vector to prepare a reproducible recombinant DNA, transforming a host by the recombinant DNA, culturing the transformant to express the DNA encoding leukocidin or proleukocidin to produce leukocidin or proleukocidin, and isolating the resulting leukocidin or pro-leukocidin.

# Fig. 1

# Fig. 2

# Fig. 3

# Fig. 4

# Fig. 5

```
        10        20        30        40        50        60        70
GAAGCTTAAATAAATATTCTCAAGTTAATAAATAATTAACTTTTAGATGGATTCATCAAAAATCGTAAAA

        80        90       100       110       120       130       140
AGAACAATTTGTATTTTACAAACATTAATTAAAAATAAAAGCAAGACATTCGTGCAATCGGTTACCTTAA

       150       160       170       180       190       200       210
ATTGTTTACAACTGTCAACAATACCAAGGTTTTATTAACTATATTTCTCACAAAATTAGCTTTTTAGCATT

       220       230       240       250       260       270       280
CCAAACAAAAAGGTTAAATTGAACGGAATTATGGCATTTTTAACTTAATTGTAAAAAAAGTTGATAATG

       290       300       310       320       330       340       350
GTCAATTGTTAATGAACAGTTAATTATAATAACGTCCAAAATATATTATTATTTAATTAAGTTAAATAAA

       360       370       380       390       400       410       420
ATTATAGAAAGACAGTGAAACTTATGCTTAAAAATAAAATATTAGCTACAACTTTATCTGTGAGCTTACT
                                 H  L  K  N  K  I  L  A  T  T  L  S  V  S  L  L

       430       440       450       460       470       480       490
TGCCCCTCTTGCCAATCCGTTATTAGAAAATGCTAAAGCTGCCAACGATACTGAAGACATCGGTAAAGGA
A  P  L  A  N  P  L  L  E  N  A  K  A  A  N  D  T  ·E  D  I  G  K  G

       500       510       520       530       540       550       560
AGCGATATAGAAATTATCAAAAGGACAGAAGATAAAACAAGTAATAAAATGGGGCGTGACTCAAAATATTC
S  D  I  E  I  I  K  R  T  E  D  K  T  S  N  K  W  G  V  T  Q  N  I  Q

       570       580       590       600       610       620       630
AATTTGATTTTGTGAAGGATACAAAATATAACAAAGATGCTCTGATATTAAAGATGCAAGGATTCATTAG
P  D  F  V  K  D  T  K  Y  N  K  D  A  L  I  L  K  M  Q  G  F  I  S

       640       650       660       670       680       690       700
CTCTAGAACAACATATTACAACTATAAAAAAACTAATCATGTTAAAGCTATGCGATGGCCATTCCAATAT
S  R  T  T  Y  Y  N  Y  K  K  T  N  R  V  K  A  M  R  W  P  F  Q  Y

       710       720       730       740       750       760       770
AATATTGGTTTAAAAACAAATGATAAATATGTTTCTTTAATTAATTATTTACCTAAAAATAAAATTGAAT
N  I  G  L  K  T  N  D  K  Y  V  S  L  I  N  Y  L  P  K  N  K  I  E  S

       780       790       800       810       820       830       840
CTACAAACGTGAGTCAGACATTAGGATACAATATCGGTGGTAATTTCCAATCAGCCCCATCACTCGGTGC
T  N  V  S  Q  T  L  G  Y  N  I  G  G  N  F  Q  S  A  P  S  L  G  G

       850       860       870       880       890       900       910
TAATGGATCATTTAACTATTCTAAATCGATTAGCTATACACAACAAAATTATGTAAGTGAAGTAGAACAA
N  G  S  P  N  Y  S  K  S  I  S  Y  T  Q  Q  N  Y  V  S  E  V  E  Q

       920       930       940       950       960       970       980
CAAAACTCAAAAAGTGTTTTATGGGGCGTCAAAGCCGAATTCATTCGCCACTGAATCAGGTCAAAAATCAG
Q  N  S  K  S  V  L  W  G  V  K  A  N  S  F  A  T  E  S  G  Q  K  S  A

       990      1000      1010      1020      1030      1040      1050
CATTTGATAGCGATTTATTTGTAGGCTACAAACCTCATAGTAAAGATCCTAGAGATTATTTCGTTCCAGA
F  D  S  D  L  F  V  G  Y  K  P  H  S  K  D  P  R  D  Y  F  V  P  D

      1060      1070      1080      1090      1100      1110      1120
CAGTGAGTTACCTCCTCTTGTACAAAGTGGATTTAACCCCTTCATTTATCGCCACAGTATCTCATGAAAAA
S  E  L  P  P  L  V  Q  S  G  F  N  P  S  F  I  A  T  V  S  H  E  K

      1130      1140      1150      1160      1170      1180      1190
GGTTCAAGCGATACAAGCGAATTTGAAATTACTTACCGGAAGAAACATGGATGTCACTCATGCCATTAAAA
G  S  S  D  T  S  E  P  E  I  T  Y  G  R  N  H  D  V  T  R  A  I  K  R

      1200      1210      1220      1230      1240      1250      1260
GATCAACGGCATTATGGCAACAGTTATTTAGACGGACATAGAGTCCATAATGCATTCGTAAATAGAAACTA
S  T  H  Y  G  N  S  Y  L  D  G  H  R  V  H  N  A  F  V  N  R  N  Y

      1270      1280      1290      1300      1310      1320      1330
TACTGTTAAATACGACGTCAATTGGAAGACTCATGAAATCAAGGTGAAAGGACAGAATTGATATCAAAAT
T  V  K  Y  E  V  H  W  K  T  H  E  I  K  V  K  G  Q  N        -

      1340      1350      1360      1370      1380      1390      1400
GAATAAAATTAGTCAAATCATCCGTTGCTACATCTATGGCATTATTATTACTTTCTGGTACTGCTAATGCT
                                                                   ──→
      1410      1420      1430      1440      1450      1460
GAGGTAAAAATAACACCACTCAGCCGTAAAAAAGTCGATCGACAAGGTTACTTTATACAAACCACCACC
    ─────────→          ◄──
```

# Fig. 6

*Fig. 7*

# Fig. 8

```
      1   2   3   4   5

97K—

66K—


45K—


31K—


21K—
14K—
```

# Fig. 9

# Fig. 10

97 K
66 K
45 K
31 K
21 K

1    2    3

# Fig. 1 1

# Fig. 12

# Fig. 13

```
      10        20        30        40        50        60        70        80        90
GAATTCATTCGCCACTGAATCAGGTCAAAAATCAGCATTTGATAGCGATTTATTTGTAGGCTACAAACCTCATAGTAAAGATCCTAGAGA
AsnSerPheAlaThrGluSerGlyGlnLysSerAlaPheAspSerAspLeuPheValGlyTyrLysProHisSerLysAspProArgAsp

      100       110       120       130       140       150       160       170       180
TTATTTCGTTCCAGACAGTGAGTTACCTCCTCTTGTACAAAGTGGATTTAAACCCTTCATTTATCGCCACAGTATCTCATGAAAAAGGTTC
TyrPheValProAspSerGluLeuProProLeuValGlnSerGlyPheAsnProSerPheIleAlaThrValSerHisGluLysGlySer

      190       200       210       220       230       240       250       260       270
AAGCGATACAAGCGAATTTGAAATTACTTACGGAAGAAACATGGATGTCACTCATCGCCATTAAAAGATCAACGCATTATGGCAACAGTTA
SerAspThrSerGluPheGluIleThrTyrGlyArgAsnMetAspValThrHisAlaIleLysArgSerThrHisTyrGlyAsnSerTyr

      280       290       300       310       320       330       340       350       360
TTTAGACGGACATAGAGTCCATAATGCATTCGTAAATAGAAACTATACTGTTAAATACGAGGTCAATTGGAAGACTCATGAAATCAAGGT
LeuAspGlyHisArgValHisAsnAlaPheValAsnArgAsnTyrThrValLysTyrGluValAsnTrpLysThrHisGluIleLysVal

SD      370       380       390       400       410       420       430       440       450
GAAAGGACAGAATTGATATGAAAATGAATAAAATTAGTCAAATCATCCGTTGCTACATCTATGGCATTATTATTACTTTCTGGTACTGCTA
LysGlyGlnAsn•••  MetLysMetAsnLysLeuValLysSerSerValAlaThrSerMetAlaLeuLeuLeuLeuSerGlyThrAlaAsn

      460       470       480       490       500       510       520       530       540
ATGCTGAAGGTAAAATAACACCAGTCAGCGTAAAAAAAGTCGATGACAAAGTTACTTTATACAAAACAACAGCCACAGCAGATTCTGATA
AlaGluGlyLysIleThrProValSerValLysLysValAspAspLysValThrLeuTyrLysThrThrAlaThrAlaAspSerAspLys

      550       560       570       580       590       600       610       620       630
AGTTTAAAATTTCACAGATTTTAACATTTAATTTCATCAAAGATAAAAGTTATGATAAAGATACTTTAGTACTTAAAGCTACTGGGAATA
PheLysIleSerGlnIleLeuThrPheAsnPheIleLysAspLysSerTyrAspLysAspThrLeuValLeuLysAlaThrGlyAsnIle

      640       650       660       670       680       690       700       710       720
TTAACTCAGGCTTTGTGAAACCTAATCCTAATGACTATGACTTTTCAAAATTATATTGGGGAGCTAAATACAATGTATCTATAAGCTCAC
AsnSerGlyPheValLysProAsnProAsnAspTyrAspPheSerLysLeuTyrTrpGlyAlaLysTyrAsnValSerIleSerSerGln

      730       740       750       760       770       780       790       800       810
AATCTAATGATTCAGTAAACGCTGTTGATTATGCACCAAAAAATCAAAATGAAGAGTTTCAAGTTCAAAATACTTTAGGCTATACATTTG
SerAsnAspSerValAsnAlaValAspTyrAlaProLysAsnGlnAsnGluGluPheGlnValGlnAsnThrLeuGlyTyrThrPheGly

      820       830       840       850       860       870       880       890       900
GTGGTGACATTAGTATCTCTAATGGTTTATCTGGTGGACTTAATGGAAATACAGCTTTTTTCTGAAACAATTAATTATAAACAAGAAAGTT
GlyAspIleSerIleSerAsnGlyLeuSerGlyGlyLeuAsnGlyAsnThrAlaPheSerGluThrIleAsnTyrLysGlnGluSerTyr

      910       920       930       940       950       960       970       980       990
ACAGAACATTAAGTCGCAACACAAATTATAAAAATGTTGGCTGGGGAGTTGAAGCACATAAAATTATGAATGGTTGGGGGACCTTATGGAA
ArgThrLeuSerArgAsnThrAsnTyrLysAsnValGlyTrpGlyValGluAlaHisLysIleMetAsnGlyTrpGlyProTyrGlyArg

      1000      1010      1020      1030      1040      1050      1060      1070      1080
GAGATAGCTTCCACCCAACATATGGTAATGAACTCTTCTTAGCTGGCAGACAAAGCAGTGCATACGCTGGCCAAAACTTCATAGCGCAAC
AspSerPheHisProThrTyrGlyAsnGluLeuPheLeuAlaGlyArgGlnSerSerAlaTyrAlaGlyGlnAsnPheIleAlaGlnHis

      1090      1100      1110      1120      1130      1140      1150      1160      1170
ACCAAATGCCATTATTATCTAGAAGTAACTTCAATCCCAGATTTTTAAGCGTACTATCACACAGACAAGATGCCGCTAAAAAATCTAAAA
GlnMetProLeuLeuSerArgSerAsnPheAsnProArgPheLeuSerValLeuSerHisArgGlnAspAlaAlaLysLysSerLysIle

      1180      1190      1200      1210      1220      1230      1240      1250      1260
TTACAGTAACTTATCAACGTGAAATGGATTTATACCAAATTCGTTGGAATGGCTTCTACTGGGCAGGCGCAAATTATAAAAAACTTTAAAA
ThrValThrTyrGlnArgGluMetAspLeuTyrGlnIleArgTrpAsnGlyPheTyrTrpAlaGlyAlaAsnTyrLysAsnPheLysThr

      1270      1280      1290      1300      1310      1320      1330      1340      1350
CTAGAACATTTAAATCAACATATGAAATTGATTGGGAAAATCACAAAGTGAAATTGTTAGATACAAAAGAAACTGAAAACAATAAATAGC
ArgThrPheLysSerThrTyrGluIleAspTrpGluAsnHisLysValLysLeuLeuAspThrLysGluThrGluAsnAsnLys•••

      1360      1370      1380      1390      1400      1410      1420      1430      1440
TAGTAAAACACGGTCGCCAACAGTAATTGTGACGACCGTGTTTTGATTTATTATCTTAGTAAGACTGCCATTCTTTTTCTCAATGTGAGA

      1450      1460      1470      1480      1490      1500      1510      1520      1530
TATAAAGGAATAGCTACAATTAAAGTGAATATTACGCCTGGAATCGCGTTTAACAACACTACCCACACAGGTAAATTTAAAATAATAGAT

      1540      1550      1560      1570      1580      1590      1600      1610      1620
AATAGTAGCCTAGATACCAAACTGCCTAATACACTTGATAAAACTAATGATAGTACATTTATTTTCAATAAATAAACAACTGCAATAGCT

      1630      1640      1650      1660      1670      1680      1690      1700      1710
ATAACTCTAAATATAATAGAAATAATCACATTAATTGGATTAAATACGCCAAATATCAGTAATAATACGCTTGATAAAAGCCCACCTAAA

      1720      1730      1740      1750      1760      1770      1780      1790      1800
AAGTACTTTTTAATTCCAAAGAAAGCTAATATCAATAATGCTGCCGGTGCAGATAAATTGAAAATCTAATCCTGGTATAATGGACGGTATT

      1810      1820      1830      1840      1850      1860      1870      1880      1890
TTCAAAACTGCCAAAATGGTTAAAAATCGCAGCAATGACACTAATTTGAGTAATATCTTTTGATGTCATACTAAAACCCCCTATACCGTTTC

      1900      1910      1920      1930      1940      1950      1960      1970      1980
ATAAACTTCTTGCTTCGGTGTGCTTTCTAAAAATGATATGTAATGATTTAAATCAATACAATCGTCCACAAATATTATTCTGCCTCCATA

      1990      2000      2010
TCTCGTATTAACTGGTTTAATATCAAATAATC
```

# Fig. 14

# Fig. 15

# Fig. 16

International application No.

PCT/JP92/01317

## A. CLASSIFICATION OF SUBJECT MATTER

Int. Cl$^5$    G01N33/569, C12P21/02, C12P21/08, C12N15/10, C12N15/31, C07K13/00//(C12N15/31, C12R1:445)

According to International Patent Classification (IPC) or to both national classification and IPC

## B. FIELDS SEARCHED

Minimum documentation searched (classification system followed by classification symbols)

Int. Cl$^5$    G01N33/569, C12P21/02, C12P21/08, C12N15/10, C12N15/31, C07K13/00

Documentation searched other than minimum documentation to the extent that such documents are included in the fields searched

Electronic data base consulted during the international search (name of data base and, where practicable, search terms used)

CAS, WPI, BIOSIS PREVIEW

## C. DOCUMENTS CONSIDERED TO BE RELEVANT

| Category* | Citation of document, with indication, where appropriate, of the relevant passages | Relevant to claim No. |
|---|---|---|
| X | Infect. Immun. Vol. 56, No. 1, 1988, Morinaga N. et al., "Changes in binding of Staphylococcal leukocidin to HL-60 cells during differentiation induced by DMSO"  p. 2479-2483 | 2, 6 |
| P,X | Biochem. Biophys. Res. Commun., Vol. 181, No. 1, 1991, Rahman A. et al., "Nucleotide sequence of leukocidin S-component gene LUKS from methicillin resistant Staphylococcus-aureus" p. 138-144 | 2-5, 10, 11 |
| P,X | Biochem. Biophys. Res. commun., Vol. 181, No. 2, 1992, Rahman A. et al., "Molecular cloning and nucleotide sequence of leukoxidin F-component gene LUKF from methicillin resistant Staphy lococcus-aureus" p. 640-646 | 6-11 |

[X] Further documents are listed in the continuation of Box C.        [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| December 10, 1992 (10. 12. 92) | January 7, 1993 (07. 01. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)